# EUROPEAN PATENT SPECIFICATION

(11) **EP 3 565 890 B1**
(45) Date of publication and mention of the grant of the patent: **01.11.2023**
(21) Application number: 18736458.3
(22) Date of filing: 05.01.2018
(51) Int. Cl.: C12N 5/0786

(54) **METHODS OF GENERATING HEPATIC MACROPHAGES AND USES THEREOF**
VERFAHREN ZUR ERZEUGUNG VON HEPATISCHEN MAKROPHAGEN UND VERWENDUNGEN DAVON
PROCÉDÉS DE GÉNÉRATION DE MACROPHAGES HÉPATIQUES ET UTILISATIONS CORRESPONDANTES

(30) Priority: 05.01.2017 SG 10201700068V
(43) Date of publication of application: 13.11.2019
(73) Proprietor: Agency for Science, Technology and Research, Singapore 138632 (SG)
(72) Inventor: TASNIM, Farah, Singapore 138669 (SG); YU, Hanry, Singapore 138669 (SG)
(74) Representative: Zwicker, Jörk
(86) International application number: PCT/SG2018/050007
(87) International publication number: WO 2018/128586

(56) References cited:
- WO-A1-99/53021
- WO-A1-2010/005594
- US-A1- 2002 151 054
- TAKEZAWA R ET AL: "Kupffer Cells Differentiation From Bone Marrow Cells By Hepatocyte-Conditioned Medium In Vitro", MOLECULAR BIOLOGY OF THE CELL, AMERICAN SOCIETY FOR CELL BIOLOGY, US, vol. 7, no. Suppl, 1 December 1996 (1996-12-01), page 305A, XP009515463, ISSN: 1059-1524
- BONNIE VAN WILGENBURG ET AL: "Efficient, Long Term Production of Monocyte-Derived Macrophages from Human Pluripotent Stem Cells under Partly-Defined and Fully-Defined Conditions", PLOS ONE, vol. 8, no. 8, 12 August 2013 (2013-08-12) , pages 1-18, XP055477903, DOI: 10.1371/journal.pone.0071098
- MAKOTO NAITO ET AL: "Development, differentiation, and maturation of Kupffer cells", MICROSCOPY RESEARCH AND TECHNIQUE., vol. 39, no. 4, 15 November 1997 (1997-11-15), pages 350-364, XP055705367, GB ISSN: 1059-910X, DOI: 10.1002/(SICI)1097-0029(19971115)39:4<350: :AID-JEMT5>3.0.CO;2-L
- TAKEZAWA R. et al.: "Kupffer Cells Differentiation From Bone Marrow Cells By Hepatocyte-Conditioned Medium In Vitro", Molecular Biology of the Cell, vol. 7 1 December 1996 (1996-12-01), page 305a, XP009515463, ISSN: 1059-1524 [retrieved on 2018-02-28]
- RIES K. et al.: "Elevated Expression of Hormone-Regulated Rat Hepatocyte Functions In A New Serum-Free Hepatocyte-Stromal Cell Coculture Model", In Vitro Cell . Dev. Biol. -Animal, vol. 36, no. 8 30 September 2000 (2000-09-30), pages 502-512, XP055299382, [retrieved on 2018-02-28]
- MURRAY P.J. et al.: "Macrophage activation and polarization: nomenclature and experimental guidelines", Immunity, vol. 41, no. 1 17 July 2014 (2014-07-17), pages 14-20, XP002776702, [retrieved on 2018-02-28]

## Description

### FIELD OF THE INVENTION

The present invention relates to cell biology and biochemistry, in particular methods of deriving and maintaining hepatic macrophages.

### BACKGROUND OF THE INVENTION

Liver is responsible for a wide variety of functions, the most important being metabolism, detoxification and protein synthesis. Many of these functions are carried out in hepatocytes, which are parenchymal cells making up 60% of all liver cells. However, the liver also contains a large proportion of non-parenchymal cells including liver sinusoidal endothelial cells, hepatic stellate cells, cholangiocytes and Kupffer cells (KCs). Kupffer cells are resident macrophages in the liver. They are located in the liver sinusoids, and are the largest population of innate immune cells in the liver, comprising approximately 10-15% of all liver cells and representing more than 80% of tissue macrophages in the body.

Owing to their abundance and localization, Kupffer cells are critical cellular components of the intrahepatic innate immune system. They are specialized in performing scavenger and phagocytic functions and playing an important role in liver immunity and in the modulation of xenobiotic metabolism during liver inflammation. Kupffer cells constitute the first macrophage population of the body to act as a barrier for pathogens and pathogen-derived products, such as lipopolysaccharide entering the liver via the portal vein. Kupffer cells play an important role in normal liver physiological homeostasis and in modulating acute and chronic responses of the liver to toxic compounds. Kupffer cells exert such effects by both direct cell-to-cell contact as well as release of a variety of inflammatory cytokines and growth factors and reactive oxygen species upon activation. These secreted factors, in particular Interleukin 6 (IL-6) and Tumor Necrosis Factor-a (TNFα) are known to induce the synthesis of acute phase proteins and suppress activities of cytochrome P450 (CYP) enzymes (the main group of metabolic enzymes involved in detoxification of xenobiotic substances). A good example to demonstrate the role of activated Kupffer cells in modulating hepatocyte injury is acetaminophen-induced hepatotoxicity. Kupffer cells have been reported to contribute to injury as well as to protect against hepatocellular damage, suggesting that Kupffer cells might be the primary site of an initially protective response which develops to cause damage upon further stimulation. Since those interactions lead to severe pharmacological and toxicological consequences, there is an evident, unmet need to develop a commercially available *in vitro* model that can mimic basal and inflammatory states of the liver. Such a system would be vital for studying drug metabolism and discovery, hepatotoxicity and predicting clinical outcomes.

Takezawa et al. ("Kupffer cells differentiation from bone marrow cells by hepatocyte-conditioned medium in vitro." MOLECLTLAR BIOLOGY OF THE CELL. Vol. 7. 8120 WOODMONT AVE, STE 750, BETHESDA, MD 20814-2755 USA: AMER SOC CELL BIOLOGY, 1996) report differentiation of unspecified bone marrow cells into Kupffer cells. Van Wilgenburg et al. ("Efficient, Long Term Production of Monocyte-Derived Macrophages from Human Pluripotent Stem Cells under Partly-Defined and Fully-Defined Conditions." PloS one 8.8 (2013): e71098) disclose production of functional monocytes and macrophages from human embryonic stem cells. The method utilises the spontaneous differentiation of hESC into embryoid bodies (comprising ectoderm, mesoderm and endoderm), followed by directed differentiation along the myeloid lineage by IL-3 and M-CSF, to produce a homogeneous population of monocytes. Naito et al. ("Development, differentiation, and maturation of Kupffer cells." Microscopy research and technique 39.4 (1997): 350-364) describe the differentiation, maturation and proliferation of Kupffer cells of mice, rat and human origin. WO 99/53021 discloses a biologically active factor capable of influencing differentiation, proliferation and/or maintenance of pluripotent cells. Specifically, a suspension culture of embryonic stem cells in primary hepatocyte conditioned medium and 50% DMEM. The conditioned medium was obtained by culturing the cells using a vessel coated with gelatin.

Most reported *in vitro* studies use Kupffer cells from animal origin. Due to interspecies variability, it would be important to study functions and effects of Kupffer cells from a human origin. However, the source of human Kupffer cells is limited due to donor availability, cost and tedious isolation procedures. Also, primary human Kupffer cells (PHKCs) cannot be maintained over extended time periods or expanded in culture to obtain larger cell numbers. Thus, there is the need for a method of deriving hepatic macrophages that resemble the characteristics of the PHKCs.

### SUMMARY OF THE INVENTION

The present invention is laid out in the accompanying claims. In a first aspect, there is provided a method of deriving hepatic macrophages from pluripotent stem cells, comprising culturing pluripotent stem cells to obtain monocytes, and culturing the monocytes in a hepatic macrophage culture medium, wherein the hepatic macrophage culture medium comprises a conditioned medium and a basal medium, wherein the conditioned medium is obtained by a method comprising: (a) culturing hepatocytes in a serum-free cell culture medium in the presence of an extracellular matrix for 1 to 7 days; and (b) isolating the supernatant at the end of the culturing process in (a).

In a second aspect, there is provided a method of deriving hepatic macrophages from induced pluripotent stem cells, comprising: (a) culturing the induced pluripotent stem cells to obtain monocytes; and (b) culturing the monocytes from (a) in a hepatic macrophage culture medium in the absence of an extracellular matrix, wherein the hepatic macrophage culture medium comprises a conditioned medium and a basal medium, wherein the conditioned medium is obtained by a method comprising: (i) culturing hepatocytes in a serum-free cell culture medium in the presence of an extracellular matrix for 1 to 7 days; and (ii) isolating the supernatant at the end of the culturing process in (i). In one example, (a) culturing pluripotent stem cells to obtain monocytes further comprises: (i) culturing pluripotent stem cells to obtain embryoid bodies; and (ii) differentiating embryoid bodies to obtain monocytes. In one example, the pluripotent stem cells are induced pluripotent stem cells. In one example, culturing the monocytes in a hepatic macrophage culture medium in the method of the first aspect comprises culturing the monocytes in the hepatic macrophage culture medium in the absence of an extracellular matrix.

In one example, the basal medium in the the hepatic macrophage culture medium is a low serum medium. In one example, the ratio of the conditioned medium and the basal medium in the hepatic macrophage culture medium is in the range of 10:1 to 1:10, or 1:1. In another example, the serum level in the low serum basal medium is at a percentage of 0.1% to 10%.

Also disclosed but not forming part of the present invention is a kit for deriving hepatic macrophages from monocytes, comprising a conditioned medium and a basal medium, wherein the conditioned medium is obtained by a method comprising: (i) culturing hepatocytes in a serum-free cell culture medium in the presence of an extracellular matrix for 1 to 7 days; and (ii) isolating the supernatant at the end of the culturing process in (i).

The kit may further comprise a cell culture device that is not coated using extracellular matrix. The basal medium in the kit can be adapted to be used as a low serum basal medium. The kit may further comprise one or more of the following supplements for the basal medium: insulin, transferrin, selenous acid, albumin, fatty acids, glutamine supplements and buffering agent.

### BRIEF DESCRIPTION OF THE DRAWINGS

The invention will be better understood with reference to the detailed description when considered in conjunction with the non-limiting examples and the accompanying drawings, in which:

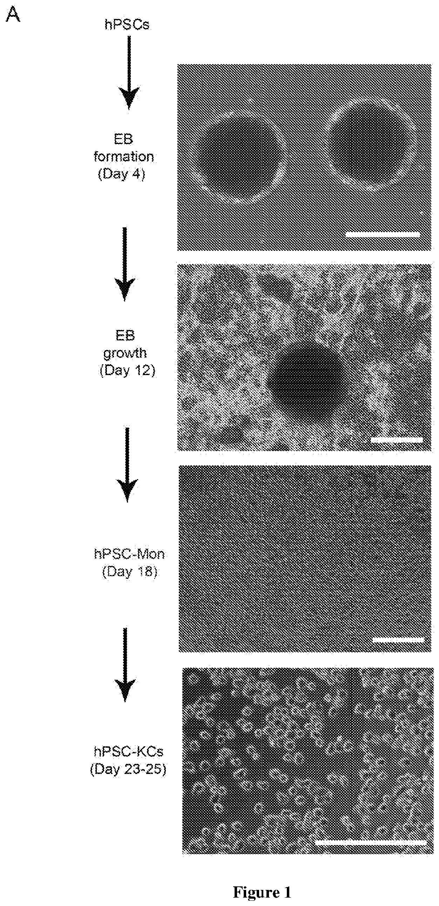

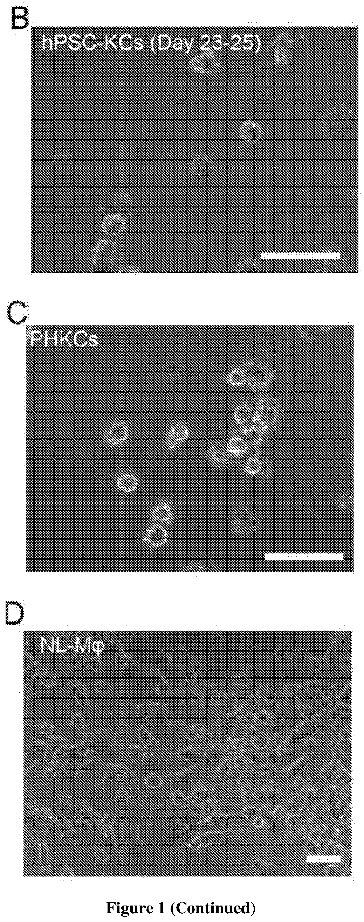

**Figure 1** shows schematics for generation of hPSC-hepatic-macrophages (hPSC-KCs) from human pluripotent stem cells (hPSCs). hPSC-Monocytes (hPSC-Mon) were derived from hPSCs and differentiated into hPSC-KCs or hPSC-non-hepatic-macrophages (NL-Mϕ), depending on the culturing conditions. **Figure 1A** shows formation and attachment of embryoid bodies (EB) and generation of non-adherent hPSC-Mon which can be harvested from the culture supernatant. hPSC-Mon were treated for additional seven days in optimized primary human hepatocyte conditioned medium to generate hPSC-KCs. Scale bar: 200 µm. **Figure 1B-**C shows phase contrast images for morphological comparison of hPSC-KCs generated using a method of the present disclosure (**1B**) to commercially available primary human kupffer cells (PHKCs) **1C.** Scale bar: 50 µm. The results show that monocytes derived from induced pluripotent stem cells could be differentiated into hepatic macrophages under suitable culturing conditions, and that the morphology of the hepatic macrophages obtained are similar to the primary human Kupffer cells. In parallel, hPSC-Mon were treated for additional seven days in X-VIVO medium containing M-CSF (no hepatic cues) to generate non-liver macrophages (NL- Mϕ). The morphology of NL-Mϕ was different from that of PHKCs and hPSC-KCs (**Figure 1C**)

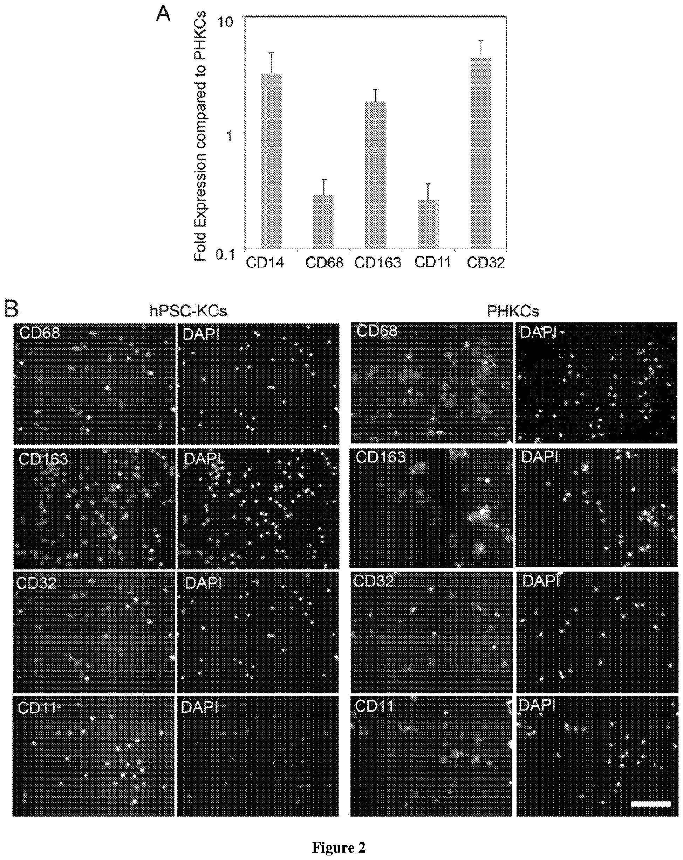

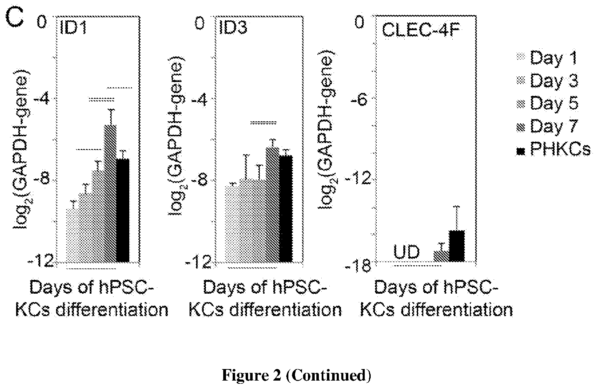

**Figure 2** shows marker expression of hPSC-KCs. In **Figure 2A****,** expression levels of the marker genes indicated on the x-axis were determined by RT-PCR and normalized to GAPDH. The expression levels hPSC-KCs were then compared to human primary kupffer cells (PHKCs). The bars show the mean ± s.d. (n = 3). In **Figure 2B****,** expression of macrophage markers in hPSC-KCs was confirmed by immunofluorescence (first panel from the left). Cells were counter-stained with DAPI (second panel from the left). Marker expression was compared to PHKCs (third panel from the left; DAPI counter stain: fourth panel from the left). Scale bar: 100 µm. The results showed hPSC-KC expressed CD14, CD163 and CD32 at levels comparable to PHKCs, while the expression levels of CD68 and CD11 are lower in hPSC-KCs as compared to PHKCs. In **Figure 2C****,** gene expression levels of KC-specific markers ID1, ID3 and CLEC-4F in hPSC-KCs at different time points during the seven day differentiation period (grey shaded bars) and PHKCs (black bars) are shown. Single solid lines represent p < 0.05 and double solid lines represent p < 0.01 (two-tailed paired t-test). Error bars represent s.e.m, n=3. UD: Undetectable. The results show that the leval of ID1, ID3 and CLEC-4F increased as the hPSC-KCs were formed during the seven day period, and that the levels of ID1, ID3 and CLEC-4F in hPSC-KCs after seven days are comparable to the levels of the same markers in PHKCs.

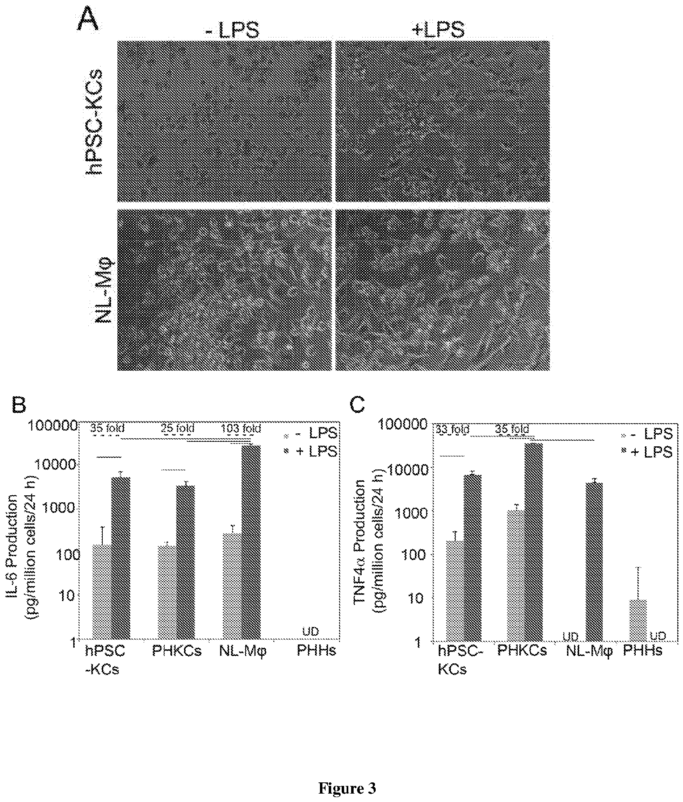

**Figure 3** shows functional similarities between PHKCs and hPSC-KCs and differences to NL-Mϕ in response to lipopolysaccharide (LPS) stimulation. **Figure 3A** are phase contrast images showing morphological changes in hPSC-KCs (upper panel) and NL-Mϕ (lower panel) following LPS treatment.. Scale bar: 100µm. **Figure 3B** shows IL-6 and **Figure 3C** shows TNF-4α production in hPSC-KCs compared to NL- Mϕ, PHKCs and primary human hepatocytes (PHHs) with (dark grey bars) and without (light grey bars) LPS treatment. Supernatant from cell cultures were collected 16 hours after LPS treatment and cytokine production was measured using ELISA. IL-6 and TNF4α (after LPS treatment) production was not detected in PHHs. TNF4α production was not detected in NL- Mϕ before LPS treatment. Data are presented after normalization to cell numbers and the bars represent mean ± s.d. (n = 3). Dashed lines represent fold differences between cytokine production before and after LPS treatment (folds are indicated on top of the dashed lines). Solid lines represent *p* < 0.05 (two-tailed paired t-test). The results show that hPSC-KCs reacted positively to LPS stimulation, in terms of morphological changes and secretion of cytokines. Importantly, the fold induction in hPSC-KCs was in the same range as that of the PHKCs (25 fold). No significant differences were observed between IL-6 levels in hPSC-KCs and PHKCs in terms of basal levels (no LPS treatment) and LPS-induced levels. The fold increase in TNF4α production in hPSC-KCs (33 fold) was similar to the fold increase in PHKCs (35 fold). In contrast to PHKCs and hPSC-KCs, NL-Mϕ showed a much higher level of LPS-induced IL-6 and TNF4α production. It has been reported that Kupffer cells show a lower extent of cytokine production upon cytokine stimulation. The results **in** **Figure 3** showed that hPSC-KCs showed LPS-induced increase in cytokine production at levels similar to that of PHKCs and these levels were much lower compared to from NL-Mϕ, confirming that they are KC-like.

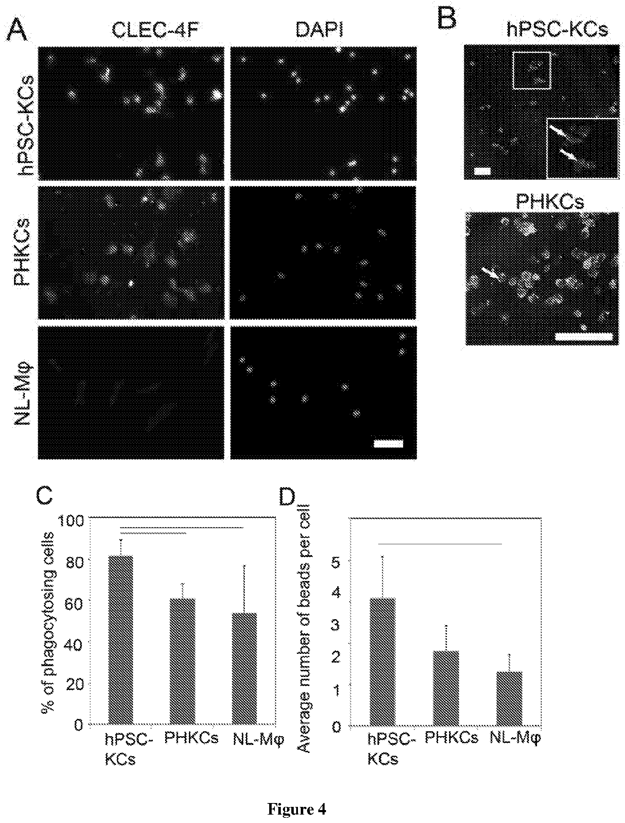

**Figure 4** shows marker and functional similarities between PHKCs and hPSC-KCs and differences to NL-Mϕ. **Figure 4A** represents immunofluorescence studies showing the expression of CLEC-4F in hPSC-KCs and PHKCs but not in NL-Mϕ. Cells were counter-stained with DAPI. Scale bar: 100µm. **Figure 4B** shows phagocytosis of carboxylate-modified 1µm FluoSpheres by hPSC-KCs and PHKCs. The white box in the right hand corner shows a magnified image of the marked section. Cells were incubated with FluoSpheres (Excitation/Emission λ= 540/560) for one hour, washed thoroughly, fixed and stained with mouse-CD163 and anti-mouse Alexa 488 (Excitation/Emission λ= 490/525) for visualization. At least ten images from each of three independent experiments were analysed and representative images are shown. Grey shading of cells represent CD163 staining and bright white dots (pointed by white arrows) show the phagocytosed beads. Confocal microscopy was used to ensure that the beads were inside the cells and not non-specific adherent beads on the cell surface. Scale bar: 50µm. **Figure 4C and D** show quantification of phagocytosis in hPSC-KCs compared to PHKCs and NL-Mϕ. At least 4 representative images from independent experiments were used for counting the number of beads uptaken and normalized to the number of cells in the respective images. Solid black lines representp < 0.05 (two-tailed paired t-test). Bars represent the mean ± s.d. The results show that hPSC-KCs but not NL-Mϕ resemble PHKCs, which are characterized by the expression of CLEC-4F, and that hPSC-KCs and PHKCs are more active in performing phagocytosis as compared to NL-Mϕ.

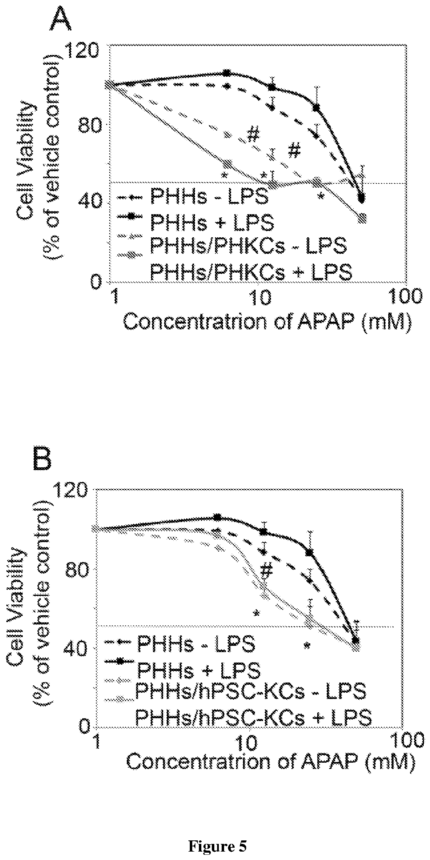

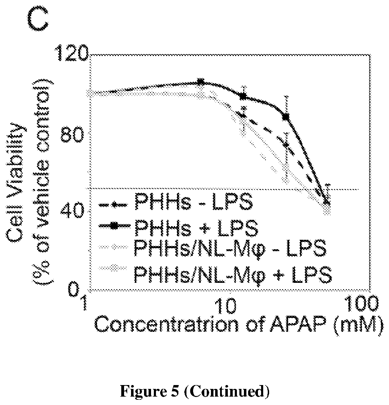

**Figure 5** shows cell toxicity response to Acetaminophen. PHHs monocultures, PHHs-PHKCs co-cultures (**Figure 5A**), PHHs-hPSC-KCs co-cultures (**Figure 5B**) and PHHs-hPSC-Mac co-cultures (**Figure 5C**) treated with LPS (solid lines) or left untreated (dashed lines) were exposed to Acetaminophen (APAP) 16 hours after LPS treatment. 24 hours after addition of the drug, cell viability was assayed using Alamar Blue. Cell viability is expressed as percentage of viable cells compared to vehicle control (DMSO). Data are presented as mean ± s.d (n=3). * indicates statistically significant differences between LPS-activated monoculture and co-culture; # indicate statistically significant differences between non-activated monoculture and co-culture (*p*<0.05). The results suggest that PHHs-hPSC-KCs represent a more sensitive model for hepatotoxicity screening as compared to PHHs/NL-Mϕ, and hPSC-KCs generated in the present study can recapitulate the response shown by commercial PHKCs when co-cultured with PHHs.

### DETAILED DESCRIPTION OF THE PRESENT INVENTION

The inventors of the present disclosure have set out to provide a method which can be used to derive hepatic macrophages. The hepatic macrophages derived resemble the characteristics of the Kupffer cells (KCs), and thus can serve as an *in vitro* model that mimics basal and inflammatory states of the liver. Further, the hepatic macrophages derived can be expanded and maintained in culture to obtain larger cell numbers. Thus, the present invention helps to circumvent the various problems associated with studies of human Kupffer cells, in particular the limited availability of primary human Kupffer cells and the inability of primary human Kupffer cells to be expanded and maintained in cell culture.

In a first aspect, there is provided a method of deriving hepatic macrophages from pluripotent stem cells, comprising culturing pluripotent stem cells to obtain monocytes, and culturing the monocytes in a hepatic macrophage culture medium, wherein the hepatic macrophage culture medium comprises a conditioned medium and a basal medium, wherein the conditioned medium is obtained by a method comprising: (a) culturing hepatocytes in a serum-free cell culture medium in the presence of an extracellular matrix for 1 to 7 days; and (b) isolating the supernatant at the end of the culturing process in (a). In one example, the method of the first aspect does not comprise the use of feeder cells.

In a second aspect, there is provided a method of deriving hepatic macrophages from induced pluripotent stem cells, comprising: (a) culturing the induced pluripotent stem cells to obtain monocytes; and (b) culturing the monocytes from (a) in a hepatic macrophage culture medium in the absence of an extracellular matrix, wherein the hepatic macrophage culture medium comprises a conditioned medium and a basal medium, wherein the conditioned medium is obtained by a method comprising: (i) culturing hepatocytes in a serum-free cell culture medium in the presence of an extracellular matrix for 1 to 7 days; and (ii) isolating the supernatant at the end of the culturing process in (i).

In one example, culturing the monocytes in a hepatic macrophage culture medium in the method of the first aspect may comprise culturing the monocytes in the hepatic macrophage culture medium in the absence of an extracellular matrix. In one example, the basal medium in the hepatic macrophage culture medium is a low serum basal medium. In one example, deriving hepatic macrophages from monocytes comprises differentiating monocytes to obtain hepatic macrophages.

The term "macrophage" as used herein refers to a type of white blood cell that engulfs and digests cellular debris, foreign substances, microbes, cancer cells, and anything else that does not have the types of proteins specific to healthy body cells on its surface, in a process called phagocytosis. Macrophages are found in essentially all tissues. They take various forms throughout the body (e.g., histiocytes, Kupffer cells, alveolar macrophages, microglia, and others), but all are part of the mononuclear phagocyte system. Besides phagocytosis, they play a critical role in nonspecific defense (innate immunity) and also help initiate specific defense mechanisms (adaptive immunity) by recruiting other immune cells such as lymphocytes. In humans, dysfunctional macrophages cause severe diseases such as chronic granulomatous disease that result in frequent infections.

Many human macrophages are produced by the differentiation of monocytes in tissues. They can be identified using flow cytometry or immuno-histochemical staining by their specific expression of proteins such as CD14, CD40, CD11b, CD64, EMR1, lysozyme M, MAC-1/MAC-3 and CD68.

The terms "hepatic macrophages" and "liver macrophages" as used interchangeably herein refer to the macrophages that resemble Kupffer cells, and which are derived using the method of the present disclosure. These macrophages are to be distinguished from the blood/bone marrow-derived macrophages. The hepatic macrophages or liver macrophages obtained using the method of the present disclosure are characterized by the expression of CLEC-4F, ID1 or ID3, or combinations thereof. CLEC-4F (C-Type Lectin Domain Family 4 Member F) is the protein encoded by the gene *CLEC-4F.* CLEC4F is a C-type lectin expressed on residential Kupffer cells. CLEC4F is not detectable in tissues other than liver. ID1 (Inhibitor Of DNA Binding 1) is the protein encoded by the gene *ID1*, and ID3 (Inhibitor Of DNA Binding 3) is the protein encoded by the gene *ID3.* Both ID1 and ID3 are helix-loop-helix (HLH) proteins that can form heterodimers with members of the basic HLH family of transcription factors. ID1 and ID3 have no DNA binding activity and therefore can inhibit the DNA binding and transcriptional activation ability of basic HLH proteins with which they interact. ID1 and ID3 have been reported as Kupffer cell specific transcription factors.

The term "Kupffer cells" as used herein refers to the self-renewing, resident and principally nonmigratory phagocyte population in the liver. Kupffer cells originate from yolk sac-derived specific progenitor cells that seed the liver during embryogenesis. Kupffer cells are highly effective phagocytes that recognize, ingest and degrade cellular debris, foreign material or pathogens. They thereby act as critical sentinels that ensure liver homeostasis and eliminate antibodies, debris or dead cells. In healthy livers, Kupffer cells are exclusively located in the intravascular compartment (mainly within the hepatic sinusoids).

The term "primary human Kupffer cells" or the short form "PHKCs" as used herein refers to Kupffer cells that are isolated from human livers, in particular adult human livers.

The term "monocyte" as used herein refers to the type of leukocyte that can differentiate into macrophages. In some examples of the present disclosure, the monocytes are derived from stem cells (i.e. stem cell-derived monocytes), in particular pluripotent stem cells, more particularly induced pluripotent stem cells, by culturing the stem cells under defined conditions. In some examples, the monocytes are obtained by (a) culturing pluripotent stem cells (in particular induced pluripotent stem cells) to obtain embryoid bodies; and (b) differentiating embryoid bodies to obtain monocytes. Such methods of obtaining monocytes from pluripotent stem cells are known in the art, for example, as described in Wilgenburg *et al.* (van Wilgenburg B, Browne C, Vowles J, Cowley SA. Efficient, long term production of monocyte-derived macrophages from human pluripotent stem cells under partly-defined and fully-defined conditions. PloS one. 2013;8:e71098) and Karlsson *et al.* (Karlsson KR, Cowley S, Martinez FO, Shaw M, Minger SL, James W. Homogeneous monocytes and macrophages from human embryonic stem cells following coculture-free differentiation in M-CSF and IL-3. Experimental hematology. 2008;36:1167-75). In some exemplary methods, the embryoid bodies are obtained from pluripotent stem cells using spin method in an embryoid bodies culture medium, followed by culturing the embryoid bodies in a differentiation medium. In some examples, the embryoid bodies are cultured in the embryoid bodies culture medium for 2 to 20 days, or 3 to 18 days, or 4 to 16 days, or 5 to 14 days, or 6 to 12 days, or 6 to 12 days, or 7 to 10 days, or 8 to 9 days, or four about 1, 2, 3, 4, 5, 6, 7, 8, 9, 10, 11, 12, 13, 14, 15, 16, 17, 18, 19 or 20 days, before being collected and cultured in a differentiation medium.

In some examples, the embryoid bodies culture medium comprises a basal medium supplemented with Rho-associated protein kinase (ROCK) inhibitor, bone morphogenetic protein, stem cell factor (SCF) and/or vascular endothelial growth factor (VEGF). One specific example of a basal medium that can be used in the embryoid bodies culture medium is mTeSR-1 medium.

The term "Rho-associated protein kinase" or the short form "ROCK" as used herein refers to kinase of the AGC (PKA/PKG/PKC) family of serine-threonine kinases. It is involved mainly in regulating the shape and movement of cells by acting on the cytoskeleton. ROCKs (ROCK1 and ROCK2) occur in mammals (human, rat, mouse, cow), zebrafish, Xenopus, invertebrates and chicken. Human ROCK1 has a molecular mass of 158 kDa and is a major downstream effector of the small GTPase RhoA. Mammalian ROCK consists of a kinase domain, a coiled-coil region and a Pleckstrin homology (PH) domain, which reduces the kinase activity of ROCKs by an autoinhibitory intramolecular fold if RhoA-GTP is not present. The term "Rho-associated protein kinase inhibitor" or the short form "ROCK inhibitor" thus refers to a compound that inhibits the activity of ROCK. Examples of ROCK inhibitors include but are not limited to Y-27632 (i.e. (R)-(+)-trans-4-(1-Aminoethyl)-N-(4-Pyridyl)cyclohexanecarboxamide dihydrochloride, IUPAC name (1R,4r)-4-((R)-1-aminoethyl)-N-(pyridin-4-yl)cyclohexanecarboxamide) and fasudil (i.e. IUPAC name 5-(1,4-Diazepane-1-sulfonyl)isoquinoline). In some examples, the ROCK inhibitor in the embryoid bodies culture medium is at a concentration of between about 1 to about 100µM, or between about 1.5 to about 90µM, or between about 2 to about 80µM, or between about 2.5 to about 70µM, or between about 3 to about 60µM, or between about 3.5 to about 50µM, or between about 4 to about 40µM, or between about 4.5 to about 30µM, or between about 5 to about 25 µM, or between about 5 to about 20µM, or between about 5 to about 15µM, or between about 5 to about 10µM, or at about 1, 2, 3, 4, 5, 6, 7, 8, 9, 10, 12.5, 15, 17.5, 20, 22.5, 25, 27.5, 30, 35, 40, 45, 50, 55, 60, 65, 70, 75, 80, 85, 90, 95 or 100µM. In one specific example, the ROCK inhibitor in the embryoid bodies culture medium is at a concentration of 10µM.

The term "bone morphogenetic protein" or the short term "BMP" as used herein refers to a group of proteins which interact with specific receptors on the cell surface, referred to as bone morphogenetic protein receptors (BMPRs). Signal transduction through BMPRs results in mobilization of members of the SMAD family of proteins. The signalling pathways involving BMPs, BMPRs and SMADs are important in the development of the heart, central nervous system, and cartilage, as well as post-natal bone development. Examples of BMPs which could be used in the embryoid bodies culture medium include but are not limited to, BMP1, BMP2, BMP3, BMP4, BMP5, BMP6, BMP7, BMP8a, BMP8b, BMP10 and BMP15. In one specific example, the BMP used is BMP4. In some examples, the BMP in the embryoid bodies culture medium is at a concentration of between about 1 to about 200 ng/ml, or between about 5 to about 180 ng/ml, or between about 10 to about 160 ng/ml, or between about 15 to about 140 ng/ml, or between about 20 to about 120 ng/ml, or between about 25 to about 100 ng/ml, or between about 30 to about 90 ng/ml, or between about 35 to about 80 ng/ml, or between about 40 to about 70 ng/ml, or between about 40 to about 60 ng/ml, or between about 40 to about 55 ng/ml, or between about 40 to about 50 ng/ml, or between about 40 to about 45 ng/ml, or at about 1, 2, 3, 4, 5, 6, 7, 8, 9, 10, 12.5, 15, 17.5, 20, 22.5, 25, 27.5, 30, 35, 40, 45, 50, 55, 60, 65, 70, 75, 80, 85, 90, 95, 100, 110, 120, 130, 140, 150, 160, 170, 180, 190 or 200ng/ml. In some specific examples, the BMP in the embryoid bodies culture medium is at a concentration of between about 1 to about 80 ng/ml. In one specific example, the BMP in the embryoid bodies culture medium is at a concentration of 50ng/ml.

The term "stem cell factor" or the short term "SCF" as used herein refers to an early-acting cytokine that plays a pivotal role in the regulation of embryonic and adult hematopoiesis. SCF promotes cell survival, proliferation, differentiation, adhesion, and functional activation of cells at multiple levels of the hematopoietic hierarchy. Together with other cytokines such as thrombopoietin and Flt3/Flk-2 Ligand, SCF is commonly used to promote expansion of primitive hematopoietic stem cells and multi-potent progenitor cells in culture. SCF exists in two biologically active splice forms: a soluble and a transmembrane isoform. Upon binding to its receptor (c-Kit tyrosine kinase receptor; CD117), it activates PI3K, JAK/STAT, and MAPK pathways. In some examples, the SCF in the embryoid bodies culture medium is at a concentration of between about 1 to about 200 ng/ml, or between about 5 to about 180 ng/ml, or between about 10 to about 160 ng/ml, or between about 15 to about 140 ng/ml, or between about 20 to about 120 ng/ml, or between about 25 to about 100 ng/ml, or between about 30 to about 90 ng/ml, or between about 35 to about 80 ng/ml, or between about 40 to about 70 ng/ml, or between about 40 to about 60 ng/ml, or between about 40 to about 55 ng/ml, or between about 40 to about 50 ng/ml, or between about 40 to about 45 ng/ml, or at about 1, 2, 3, 4, 5, 6, 7, 8, 9, 10, 12.5, 15, 17.5, 20, 22.5, 25, 27.5, 30, 35, 40, 45, 50, 55, 60, 65, 70, 75, 80, 85, 90, 95, 100, 110, 120, 130, 140, 150, 160, 170, 180, 190 or 200ng/ml. In some specific examples, the SCF in the embryoid bodies culture medium is at a concentration of between about 1 to about 50 ng/ml. In one specific example, the SCF in the embryoid bodies culture medium is at a concentration of 20ng/ml.

The term "vascular endothelial growth factor" or the short term "VEGF" as used herein refers to is a signal protein produced by cells that stimulates the formation of blood vessels. The VEGF family comprises in mammals five members: VEGF-A, placenta growth factor (PGF), VEGF-B, VEGF-C and VEGF-D. They are important signalling proteins involved in both vasculogenesis and angiogenesis. In some examples, the VEGF in the embryoid bodies culture medium is at a concentration of between about 1 to about 200 ng/ml, or between about 5 to about 180 ng/ml, or between about 10 to about 160 ng/ml, or between about 15 to about 140 ng/ml, or between about 20 to about 120 ng/ml, or between about 25 to about 100 ng/ml, or between about 30 to about 90 ng/ml, or between about 35 to about 80 ng/ml, or between about 40 to about 70 ng/ml, or between about 40 to about 60 ng/ml, or between about 40 to about 55 ng/ml, or between about 40 to about 50 ng/ml, or between about 40 to about 45 ng/ml, or at about 1, 2, 3, 4, 5, 6, 7, 8, 9, 10, 12.5, 15, 17.5, 20, 22.5, 25, 27.5, 30, 35, 40, 45, 50, 55, 60, 65, 70, 75, 80, 85, 90, 95, 100, 110, 120, 130, 140, 150, 160, 170, 180, 190 or 200ng/ml. In some specific examples, the VEGF in the embryoid bodies culture medium is at a concentration of between about 1 to about 80 ng/ml. In one specific example, the VEGF in the embryoid bodies culture medium is at a concentration of 50ng/ml.

In some other examples, the differentiation medium comprises a basal medium supplemented with macrophage colony-stimulating factor (M-CSF), interleukin, glutamine supplement, antioxidant and/or antibiotic. One specific example of a basal medium that can be used in the differentiation medium is X-VIVO medium.

The term "macrophage colony-stimulating factor" or the short form "M-CSF" as used herein refers to a secreted hematopoietic growth factor that is involved in the proliferation, differentiation, and survival of monocytes, macrophages, and bone marrow progenitor cells. It binds to the colony stimulating factor 1 receptor. In some examples, the M-CSF in the differentiation medium is at a concentration of between about 1 to about 500 ng/ml, or between about 10 to about 450 ng/ml, or between about 20 to about 400 ng/ml, or between about 30 to about 350 ng/ml, or between about 40 to about 300 ng/ml, or between about 50 to about 250 ng/ml, or between about 60 to about 200 ng/ml, or between about 70 to about 180 ng/ml, or between about 80 to about 160 ng/ml, or between about 90 to about 140 ng/ml, or between about 100 to about 120 ng/ml, or at about 5, 10, 15, 20, 25, 30, 35, 40, 45, 50, 55, 60, 65, 70, 75, 80, 85, 90, 95, 100, 110, 120, 130, 140, 150, 160, 170, 180, 190, 200, 220, 240, 260, 280, 300, 320, 340, 360, 380, 400, 420, 440, 460, 480 or 500ng/ml. In some specific examples, the M-CSF in the differentiation medium is at a concentration of between about 50 to about 200 ng/ml. In one specific example, the M-CSF in the differentiation medium is at a concentration of 100ng/ml.

The term "interleukin" as used herein refers to a group of cytokines that were first seen to be expressed by white blood cells. Common families of interleukins include Interleukin 1 (IL-1) to Interleukin 15 (IL-15) and Interleukin 17 (IL-17). In one specific example, the interleukin used in the differentiation medium is Interleukin 3 (IL-3). In some examples, the interleukin in the differentiation medium is at a concentration of between about 1 to about 200 ng/ml, or between about 5 to about 180 ng/ml, or between about 10 to about 160 ng/ml, or between about 15 to about 140 ng/ml, or between about 20 to about 120 ng/ml, or between about 25 to about 100 ng/ml, or between about 30 to about 90 ng/ml, or between about 35 to about 80 ng/ml, or between about 40 to about 70 ng/ml, or between about 40 to about 60 ng/ml, or between about 40 to about 55 ng/ml, or between about 40 to about 50 ng/ml, or between about 40 to about 45 ng/ml, or at about 1, 2, 3, 4, 5, 6, 7, 8, 9, 10, 12.5, 15, 17.5, 20, 22.5, 25, 27.5, 30, 35, 40, 45, 50, 55, 60, 65, 70, 75, 80, 85, 90, 95, 100, 110, 120, 130, 140, 150, 160, 170, 180, 190 or 200ng/ml. In some specific examples, the interleukin in the differentiation medium is at a concentration of between about 1 to about 50 ng/ml. In one specific example, the interleukin in the differentiation medium is at a concentration of 25ng/ml.

Glutamine supports the growth of cells that have high energy demands and synthesize large amounts of proteins and nucleic acids. It is an alternative energy source for rapidly dividing cells and cells that use glucose inefficiently. Cells require nitrogen atoms to build molecules such as nucleotides, amino acids, amino-sugars and vitamins. Ammonium is an inorganic source of nitrogen that exists primarily as a positively charged cation, NH4+, at physiological pH. Ammonium nitrogen used by cells is initially incorporated into organic nitrogen as an amine of glutamate or an amide of glutamine. These two amino acids provide the primary reservoirs of nitrogen for the synthesis of proteins, nucleic acids and other nitrogenous compounds. Examples of glutamine supplements include L-glutamine and L-alanyl-L-glutamine dipeptide. In some examples, the glutamine supplement in the differentiation medium is at a concentration of between about 0.5 to about 50mM, or between about 1 to about 45mM, or between about 1.5 to about 40mM, or between about 2 to about 35mM, or between about 3 to about 30mM, or between about 4 to about 25mM, or between about 5 to about 20mM, or between about 6 to about 18mM, or between about 7 to about 16mM, or between about 8 to about 14mM, or between about 9 to about 12mM, or between about 10 to about 11mM, or at about 0.5, 1, 1.5, 2, 2.5, 3, 3.5, 4, 4.5, 5, 6, 7, 8, 9, 10, 11, 12, 13, 14, 15, 16, 17, 18, 19, 20, 22, 24, 26, 28, 30, 35, 40, 45 or 50mM. In some specific examples, the glutamine supplement in the differentiation medium is at a concentration of between about 1 to about 10mM. In one specific example, the glutamine supplement in the differentiation medium is at a concentration of 2mM.

In one example, the antioxidant is, but not limited to, β-mercaptoethanol. In some examples, the antioxidant in the differentiation medium is at a concentration of between about 0.1 to about 10mM, or between about 0.2 to about 9mM, or between about 0.3 to about 8mM, or between about 0.4 to about 7mM, or between about 0.5 to about 6mM, or between about 0.6 to about 5mM, or between about 0.7 to about 4.5mM, or between about 0.8 to about 4mM, or between about 0.9 to about 3.5mM, or between about 1 to about 3mM, or between about 1.1 to about 2.5mM, or between about 1.2 to about 2mM, or between about 1.3 to about 1.9mM, or between about 1.4 to about 1.8mM, or between about 1.5 to about 1.7mM, or at about 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.5, 4, 4.5, 5, 6, 7, 8, 9 or 10mM. In some specific examples, the antioxidant in the differentiation medium is at a concentration of between about 0.1 to about 1mM. In one specific example, the antioxidant in the differentiation medium is at a concentration of 0.055mM.

In some examples, the antibiotic is, but is not limited to, penicillin, streptomycin, ampicillin, chloramphenicol, gentamycin, kanamycin, neomycin, tetracycline, polymyxin B, actinomycin, bleomycin, cyclohexamide, geneticin (G148), hygromycin B, mitomycin C and combinations thereof. In one example, the antibiotic is penicillin. In another example, the antibiotic is streptomycin. In yet another example, the antibiotic is penicillin and streptomycin. In some examples, the penicillin in the differentiation medium is at a concentration of between about 1 to about 500 U/ml, or between about 10 to about 450 U/ml, or between about 20 to about 400 U/ml, or between about 30 to about 350 U/ml, or between about 40 to about 300 U/ml, or between about 50 to about 250 U/ml, or between about 60 to about 200 U/ml, or between about 70 to about 180 U/ml, or between about 80 to about 160 U/ml, or between about 90 to about 140 U/ml, or between about 100 to about 120 U/ml, or at about 5, 10, 15, 20, 25, 30, 35, 40, 45, 50, 55, 60, 65, 70, 75, 80, 85, 90, 95, 100, 110, 120, 130, 140, 150, 160, 170, 180, 190, 200, 220, 240, 260, 280, 300, 320, 340, 360, 380, 400, 420, 440, 460, 480 or 500 U/ml. In some specific examples, the penicillin in the differentiation medium is at a concentration of between about 50 to about 200 U/ml. In one specific example, the penicillin in the differentiation medium is at a concentration of 100 U/ml. In some examples, the streptomycin in the differentiation medium is at a concentration of between about 1 to about 500mg/ml, or between about 10 to about 450mg/ml, or between about 20 to about 400 mg/ml, or between about 30 to about 350 mg/ml, or between about 40 to about 300 mg/ml, or between about 50 to about 250 mg/ml, or between about 60 to about 200 mg/ml, or between about 70 to about 180 mg/ml, or between about 80 to about 160 mg/ml, or between about 90 to about 140 mg/ml, or between about 100 to about 120 mg/ml, or at about 5, 10, 15, 20, 25, 30, 35, 40, 45, 50, 55, 60, 65, 70, 75, 80, 85, 90, 95, 100, 110, 120, 130, 140, 150, 160, 170, 180, 190, 200, 220, 240, 260, 280, 300, 320, 340, 360, 380, 400, 420, 440, 460, 480 or 500 mg/ml. In some specific examples, the penicillin in the differentiation medium is at a concentration of between about 50 to about 200 mg/ml. In one specific example, the penicillin in the differentiation medium is at a concentration of 100 mg/ml.

The monocytes to be cultured in the hepatic macrophage culture medium to obtain hepatic macrophages may be characterized by the expression of CD14 cell surface receptor.

The term "CD14" as used herein refers to the protein encoded by the gene *CD14* (cluster of differentiation 14). CD14 is a component of the innate immune system. It acts as a co-receptor (along with the Toll-like receptor TLR 4 and MD-2) for the detection of bacterial lipopolysaccharide (LPS). CD14 can bind LPS only in the presence of lipopolysaccharide-binding protein (LBP). Although LPS is considered its main ligand, CD14 also recognizes other pathogen-associated molecular patterns such as lipoteichoic acid.

The term "stem cell" as used herein refers to undifferentiated biological cells that are capable of differentiating into more specialized cells and that are capable of dividing (through mitosis) to produce more stem cells. Stem cells are found in multicellular organisms. In mammals, there are two broad types of stem cells: embryonic stem cells, which are isolated, for example, from the inner cell mass of non-human blastocysts and adult stem cells, which are found in various tissues. The stem cells may also be classified as pluripotent stem cells and multipotent stem cells.

The term "pluripotent stem cells" or "pluripotent cells" as used herein refers to stem cells that have complete differentiation versatility, that are self-renewing, and can remain dormant or quiescent within tissue. They have the potential to differentiate into any of the three germ layers: the endoderm, from which, for example, the interior stomach lining, gastrointestinal tract and the lungs develop; the mesoderm, from which, for example, muscle, bone, blood and urogenital structures develop; or the ectoderm, from which, for example, epidermal tissues and nervous system develop. It is noted however, that cell pluripotency is considered to be a continuum, ranging from the completely pluripotent cell that can form every cell of the embryo proper, for example, embryonic stem cells and induced pluripotent stem cells, to the incompletely or partially pluripotent cell that can form cells of all three germ layers but that may not exhibit all the characteristics of completely pluripotent cells.

The term "embryonic stem cells" as used herein refers to the pluripotent stem cells derived from the inner cell mass of a non-human blastocyst, an early-stage pre-implantation embryo. In one example, the method as disclosed herein is performed without the use of human embryonic stem cells. In yet another example, the method as disclosed herein is performed using embryonic stem cells that are not of human origin.

The term "induced pluripotent stem cells" or the short form "iPSCs" or "iPS cells" as used herein refers to pluripotent stem cells that can be generated directly from adult cells, typically adult somatic cells, for example fibroblasts, lung or B cells. iPSCs are typically derived by introducing products of specific sets of pluripotency-associated genes, or "reprogramming factors", into a given cell type. Upon introduction of reprogramming factors, cells begin to form colonies that resemble pluripotent stem cells, which can be isolated based on their morphology, conditions that select for their growth, or through expression of surface markers or reporter genes. Examples of induced pluripotent stem cells include but are not limited to, induced pluripotent stem cells generated from the human fibroblasts. In one specific example, the induced pluripotent stem cells are iPSC-IMR90 cells.

The term "multipotent stem cells" as used herein refers to unspecialized cells that have the ability to self-renew for long periods of time and differentiate into specialized cells with specific functions. A multipotent stem cell can give rise to other types of cells but it is limited in its ability to differentiate, as compared to pluripotent stem cells. Hence, adult stem cells are generally considered as multipotent stem cells because their specialization potential is limited to one or more cell lines. Examples of multipotent stem cells include, but are not limited to, hematopoietic stem cells (adult stem cells) from the bone marrow that give rise to red blood cells, white blood cells, and platelets; mesenchymal stem cells (adult stem cells) from the bone marrow that give rise to stromal cells, fat cells, and types of bone cells; epithelial stem cells that give rise to the various types of skin cells; and muscle satellite cells that contribute to differentiated muscle tissue.

Although the method of the present disclosure was demonstrated to work for the derivation of human hepatic macrophages, it is to be understood that the method could also be used for the derivation of hepatic macrophages of other mammalian species. As such, the stem cells may be isolated from any mammalian species, for example, but not limited to, mouse, rat, rabbit, guinea pig, dog, cat, pig, sheep, cow, horse, monkey and human, depending on the species that the hepatic macrophages are to be generated for. In one example, the stem cells are obtained from a human, in order to derive hepatic macrophages that resemble primary human Kupffer cells.

The term "embryoid body" or the short form "EB" refers to three-dimensional aggregates of stem cells. In contrast to monolayer cultures, the spheroid structures that are formed when stem cells aggregate enables the non-adherent culture of EBs in suspension, making EB cultures inherently scalable. This is useful for bioprocessing approaches, whereby large yields of cells can be produced for potential clinical applications.

The term "conditioned medium" as used herein refers to a cell culture medium that that is further supplemented with soluble factors derived from the cells that were used to condition the medium. For example, when hepatocytes are used to condition the medium, the conditioned medium obtained will contain soluble factors secreted by hepatocytes. Non-limiting examples of soluble factors secrete by hepatocytes include: albumin, C-reactive protein (CRP), alpha feto protein (AFP), transferrin, plasminogen, bile (which comprises organic molecules including bile acids, cholesterol, phospholipids and bilirubin), innate immunity proteins listed in Table 1, chemokines (e.g. MCP-1 and CXCL1) to attract innate immune cells and anti-inflammatory proteins such as peptidoglycan recognition proteins.

**Table 1. Innate immunity proteins secreted by hepatocytes**

| **Innate Immunity proteins** | | **Mainly synthesized in hepatocytes** | **Function** |
|---|---|---|---|
| Cs | Classical | C1r/s, C2, C4, C4bp | Activate C classical pathway |
| | Alternative | C3, B | Activate C alternative pathway |
| | Lectin | MBL, MASP1, 23, MAp19 | Activate C MBL pathway |
| | Terminal | C5, C6, C8, C9 | Terminal C components |
| | Regulators | I, H, C1-INH | Inhibit C activation |
| Opsonins | Pentraxins | CRP, SAP | Bind to microbes and subsequently activate Cs and phagocytosis to kill microbes |
| | SAA | SAA | Binds to the outer membrane protein A family members on bacteria to activate phagocytosis |
| LPS signaling regulators | Lipid transferase | LBP | Binds to LPS and subsequently transfers LPS to a receptor complex (TLR4/MD-2) via a CD14-enhanced mechanism |
| | sCD 14 | Soluble CD14 | Stimulates or inhibits LPS signaling depending on its concentration and environment |
| | sMD-2 | Soluble MD-2 | Stimulates or inhibits LPS signaling depending on its concentration and environment |
| Iron metabolism -related proteins | Iron-carrying protein | Transferrin | Binds to free iron, limiting iron availability to pathogens |
| | Lipocalin-2 | Lipocalin-2 | Attenuates iron uptake by bacteria via binding to siderophores |
| | Anti-microbial peptide | Hepcidin (also LEAP) | Anti-microbial peptide via limiting iron availability |
| | Hemopexin | Hemopexin | Retains heme from the bacteria by binding to heme |
| Others | Clotting factors | Fibrinogen | A central regulator of the inflammatory response |
| | PGRPs | PGLYP2 | Anti-inflammatory response via digestion of peptidoglycan on the bacterial wall |
| | Proteinase inhibitors | AAT, ACT, α₁-CPI, α₂M | Inactivate proteases released by pathogens and dead or dying cells |
| α₁-CPI, α₁-cysteine proteinase inhibitor (thiostain); α₂M, α₂-macroglobulin; AAT, antitrypsin; ACT, antichymotrypsin; B, factor B; C1-INH, C1 inhibitor; CRP, C-reactive protein; Cs, complements;, I, factor I; H, factor H; LBP, LPS-binding protein; LEAP, liver expressed antimicrobial peptide; MBL, mannan-binding lectin; MASP, mannan-binding lectin-associated serine proteases; PGRPs, peptidoglycan-recognition proteins; PGLYP2, peptidoglycan-recognition protein-2; SAA, serum amyloid A; SAP, serum amyloid P. | | | |

The cell culture medium that is used to obtain the conditioned medium may be a basal medium. In some examples, the basal medium is adapted to culture the cells that are used to condition the medium. In one specific example, the basal medium is adapted to culture hepatocytes, i.e. a hepatocyte specific medium. In one specific example, the basal medium is William's E medium. The basal medium used to obtain the conditioned medium could be supplemented with one or more growth supplement and/or growth enhancer as described below.

Conditioning the medium may comprise culturing the cells in the basal medium for a period of time, until the cells have completed a number of cell cycles, or until the cells have reached a specific phase in the cell cycle. For example, the cells used the condition the medium may have completed 1 cell cycle, or 2, 3, 4, 5, 6, 7, 8, 9 or 10 cell cycles. The cells may also be in the interphase Gi, S or G₂, or in the mitosis phase. In some examples, conditioning the medium comprises culturing the cells in the basal medium for about 12, 18, 24, 30, 36, 42, 48, 54, 60, 72, 84 or 96 hours, or for about 1, 2, 3, 4, 5 , 6, 7, 8, 9 or 10 days. In some examples, the cells are cultured for about 12 hours to about 10 days, or for about 1 day to about 9 days, or for about 2 days to about 8 days, or for about 3 days to about 7 days, or for about 4 days to about 6 days. In some specific examples, the cells are cultured for about 1 day to about 7 days. In one specific example, the cells are cultured for about 1 day. To obtain the conditioned medium, the supernatant is collected at the end of the culturing process. In some examples, after the supernatant is collected, a fresh medium may be used to continue culturing the cells for the period of time as defined above, so as to continue producing the conditioned medium.

In one example, condition the medium does not comprise the use of feeder cells.

The term "basal medium" as used herein refers to a cell growth medium that only contains the essential supplements for cell growth. In most cases, a basal medium that can be used for the method of the present disclosure is commercially available. Examples of commercially available basal medium include Advanced Dulbecco's Modified Eagle Medium (Advanced DMEM), DMEM, RPMI (Roswell Park Memorial Institute) 1640 medium, X-VIVO^{™} medium (a hematopoietic medium) and William's E medium. In one specific example, the basal medium used to obtain the conditioned medium is William's E medium.

In some examples, the basal medium used in the hepatic macrophage culture medium comprises one or more or all of the following components: (1) a lipid-rich albumin, for example, a lipid-rich bovine serum albumin. Specific examples of such a lipid-rich albumin include but are not limited to AlbuMax^{®} I and AlbuMax^{®} II supplied by ThermoFisher Scientific. In some examples, the concentration of the lipid-rich albumin in the basal medium used in the hepatic macrophage culture is between about 80 to about 2000mg/L, or between about 100 to about 1900mg/L, or between about 200 to about 1800mg/L, or between about 300 to about 1700mg/L, or between about 400 to about 1600mg/L, or between about 500 to about 1500mg/L, or between about 600 to about 1400mg/L, or between about 700 to about 1300mg/L, or between about 800 to about 1200mg/L, or between about 900 to about 1100mg/L, or at about 100, 150, 250, 350, 450, 550, 650, 750, 850, 950, 1050, 1150, 1250, 1350, 1450, 1550, 1650, 1750, 1850, or 1950 mg/L. In one specific example, the lipid-rich albumin is AlbuMax^{®} II at a concentration of 400mg/L. (2) A vanadate compound, for example, ammonium metavanadate. The vanadate compound is usually added in trace amount to the basal medium, for example, at a concentration of between about 6×10⁻⁵ to about 1.5×10⁻³mg/L, or between about 1×10⁻⁴ to about 1.4×10⁻³mg/L, or between about 2×10⁻⁴ to about 1.3×10⁻³mg/L, or between about 3×10⁻⁴ to about 1.2×10⁻³ mg/L, or between about 4×10⁻⁴ to about 1.1×10⁻³ mg/L, or between about 5×10⁻⁴ to about 1×10⁻³mg/L, or between about 6×10⁻⁴ to about 9×10⁻⁴mg/L, or between about 7×10⁻⁴ to about 8×10⁻⁴mg/L, or at about 8×10⁻⁵, 1×10⁻⁴, 1.5×10⁻⁴, 2.5×10⁻⁴, 3.5×10⁻⁴, 4.5×10⁻⁴, 5.5×10⁻⁴, 6.5×10⁻⁴, 7.5×10⁻⁴, 8.5×10⁻⁴, 9.5×10⁻⁴, 1×10⁻³, 1.05×10⁻³, 1.15×10⁻³, 1.25×10⁻³, 1.35×10⁻³, or 1.45×10⁻³mg/L. In one specific example, the vanadate compound is ammonium metavanadate at a concentration of 3×10⁻⁴mg/L. (3) Vitamin C (also known as ascorbic acid or L-ascorbic acid) or its derivative. Examples of vitamin C derivative include but are not limited to ascorbic acid phosphate (in particular ascorbic acid 2-phosphate), sodium ascorbyl phosphate and magnesium ascorbyl phosphate. In some examples, the concentration of vitamin C or its derivative in the basal medium used in the hepatic macrophage culture is between about 0.5mg/L to about 12.5mg/L, or between about 1mg/L to about 12mg/L, or between about 1.5mg/L to about 11.5mg/L, or between about 2mg/L to about 11mg/L, or between about 2.5mg/L to about 10.5mg/L, or between about 3mg/L to about 10mg/L, or between about 3. 5mg/L to about 9. 5mg/L, or between about 4mg/L to about 9mg/L, or between about 4.5mg/L to about 8.5mg/L, or between about 5mg/L to about 8mg/L, or between about 5.5mg/L to about 7.5mg/L, or between about 6mg/L to about 7mg/L, or at about 0.5, 0.75, 1.25, 1.75, 2.25, 2.75, 3.25, 3.75, 4.25, 4.75, 5.25, 5.75, 6.25, 6.75, 7.25, 7.75, 8.25, 8.75, 9.25, 9.75, 10.25, 10.75, 11.25, 11.75, or 12.25 mg/L. In one specific example, ascorbic acid phosphate at a concentration of 2.5mg/L is used in the basal medium that is used in the hepatic macrophage culture medium. (4) A calcium supplement, preferably in soluble form. One specific example of such a calcium supplement is calcium chloride, the anhydrous or dihydrate form of which can be used. In some examples, the concentration of the calcium supplement in the basal medium used in the hepatic macrophage culture is between about 40mg/L to about 1000mg/L, or between about 100mg/L to about 900mg/L, or between about 150mg/L to about 950mg/L, or between about 200mg/L to about 900mg/L, or between about 250mg/L to about 850mg/L, or between about 300mg/L to about 800mg/L, or between about 350mg/L to about 750mg/L, or between about 400mg/L to about 700mg/L, or between about 450mg/L to about 650mg/L, or between about 500mg/L to about 600mg/L, or at about 80, 125, 175, 225, 275, 325, 375, 425, 475, 525, 575, 625, 675, 725, 775, 825, 875, 925, 975, or 1000mg/L. In one specific example, the calcium supplement used in the basal medium that is used in the hepatic macrophage culture medium is calcium chloride in anhydrous form at a concentration of 200mg/L. (5) A copper supplement, preferably in soluble form. Specific examples of copper supplement include but are not limited to copper (II) sulphate (also known as cupric sulphate) and copper (II) chloride. In some examples, the concentration of the copper supplement in the basal medium used in the hepatic macrophage culture is between about 0.25×10⁻³mg/L to about 6.25×10⁻³ mg/L, or between about 0.5×10⁻³ mg/L to about 6×10⁻³mg/L, or between about 1×10⁻³mg/L to about 5.5×10⁻³mg/L, or between about 1.5×10⁻³mg/L to about 5×10⁻³mg/L, or between about 2×10⁻³mg/L to about 4.5×10⁻³mg/L, or between about 2.5×10⁻³ mg/L to about 4×10⁻³mg/L, or between about 3×10⁻³mg/L to about 3.5×10⁻³mg/L, or at about 0.25×10⁻³, 0.75×10⁻³, 1.25×10⁻³, 1.75×10⁻³, 2.25×10⁻³, 2.75×10⁻³ , 3.25×10⁻³ , 3.75×10⁻³ , 4.25×10⁻³, 4.75×10⁻³, 5.25×10⁻³, 5.75×10⁻³, or 6.25×10⁻³mg/L. In one specific example, the copper supplement used in the basal medium that is used in the hepatic macrophage culture medium is cupric sulfate at a concentration of 1.25× 10⁻³mg/L. (6) Vitamin B₅ (also known as pantothenic acid) or its derivative. Examples of vitamin B₅ derivative include but are not limited to calcium pantothenate, in particular D-calcium pantothenate, and provitamin panthenol (also known as pantothenol). In some examples, the concentration of vitamin B₅ or its derivative in the basal medium used in the hepatic macrophage culture is between about 0.8mg/L to about 16mg/L, or between about 1mg/L to about 15mg/L, or between about 2mg/L to about 14mg/L, or between about 3mg/L to about 13mg/L, or between about 4mg/L to about 12mg/L, or between about 5mg/L to about 11mg/L, or between about 6mg/L to about 10mg/L, or between about 7mg/L to about 9mg/L, or at about 1.5, 2.5, 3.5, 4.5, 5.5, 6.5, 7.5, 8.5, 9.5, 10.5, 11.5, 12.5, 13.5, 14.5, or 15.5mg/L. In one specific example, D-calcium pantothenate at a concentration of 4mg/L is used in the basal medium that is used in the hepatic macrophage culture medium. (7) Ethanolamine. In some examples, the concentration of ethanolamine in the basal medium used in the hepatic macrophage culture is between about 0.3mg/L to about 10mg/L, or between about 0.5mg/L to about 9.5mg/L, or between about 1mg/L to about 9mg/L, or between about 1.5mg/L to about 8.5mg/L, or between about 2mg/L to about 8mg/L, or between about 2.5mg/L to about 7.5mg/L, or between about 3mg/L to about 7mg/L, or between about 3.5mg/L to about 6.5mg/L, or between about 4mg/L to about 6mg/L, or between about 4.5mg/L to about 5.5mg/L, or at about 0.25, 0.75, 1.25, 1.75, 2.25, 2.75, 3.25, 3.75, 4.25, 4.75, 5.25, 5.75, 6.25, 6.75, 7.25, 7.75, 8.25, 8.75, 9.25, or 9.75mg/L. In one specific example, the concentration of ethanolamine in the basal medium used in the hepatic macrophage culture is 1.9mg/L. (8) An iron supplement, preferably in soluble form. In some examples, the iron supplement is a ferric iron (i.e. Iron (III)) supplement, for example, ferric nitrate. In some other example, the iron supplement is a ferrous iron (i.e. Iron (II)) supplement, for example, ferrous sulfate. In one specific example, the ferric nitrate used is in the form of ferric nitrate nonahydrate (Fe(NO₃)₃•9H₂O). In another specific example, the ferrous sulfate use is in the form of ferrous sulfate heptahydrate (FeSO₄•7H₂O). In some examples, the concentration of iron supplement in the basal medium used in the hepatic macrophage culture is between about 0.02mg/L to about 0.5mg/L, or between about 0.05mg/L to about 0.45mg/L, or between about 0.1mg/L to about 0.4mg/L, or between about 0.15mg/L to about 0.35mg/L, or between about 0.2mg/L to about 0.3mg/L, or at about 0.02, 0.05, 0.08, 0.1, 0.125, 0.15, 0.175, 0.2, 0.225, 0.25, 0.275, 0.3, 0.325, 0.35, 0.375, 0.4, 0.425, 0.45, 0.475, or 0.5mg/L. In one specific example, ferric nitrate nonahydrate at a concentration of 0.1mg/L is used in the basal medium that is used in the hepatic macrophage culture medium. (9) Inositol, also known as cyclohexane-1,2,3,4,5,6-hexol. In one specific example, the inositol used is myo-Inositol, also known as 1,2,3,4,5,6-hexahydroxycyclohexane, i-Inositol or meso-Inositol. Myo-Inositol plays an important role as the structural basis for a number of secondary messengers in eukaryotic cells. In addition, inositol serves as an important component of the structural lipids phosphatidylinositol (PI) and its various phosphates - the phosphatidylinositol phosphate (PIP) lipids. In some examples, the concentration of Inositol in the basal medium used in the hepatic macrophage culture is between about 1mg/L to about 40mg/L, or between about 5mg/L to about 35mg/L, or between about 7.5mg/L to about 30mg/L, or between about 10mg/L to about 25mg/L, or between about 12.5mg/L to about 20mg/L, or between about 15mg/L to about 17.5mg/L, or at about 1, 2.5, 5, 8, 10, 12, 14, 16, 18, 20, 22, 24, 26, 28, 30, 32, 34, 36, 38 or 40mg/L. In one specific example, myo-Inositol at a concentration of 7.2mg/L is used in the basal medium that is used in the hepatic macrophage culture medium. (10) A carbon supplement. In one example, the carbon supplement is sodium pyruvate, which is commonly provided as a carbon source in addition to glucose. In some examples, the concentration of the carbon supplement in the basal medium used in the hepatic macrophage culture is between about 20mg/L to about 600mg/L, or between about 30mg/L to about 550mg/L, or between about 40mg/L to about 500mg/L, or between about 50mg/L to about 450mg/L, or between about 60mg/L to about 400mg/L, or between about 70mg/L to about 350mg/L, or between about 80mg/L to about 300mg/L, or between about 90mg/L to about 250mg/L, or between about 100mg/L to about 200mg/L, or between about 110mg/L to about 190mg/L, or between about 120mg/L to about 180mg/L, or between about 130mg/L to about 170mg/L, or between about 140mg/L to about 160mg/L, or at about 25, 50, 75, 100, 125, 150, 175, 200, 225, 250, 275, 300, 325, 350, 375, 400, 425, 450, 475, 500, 525, 550, 575 or 600mg/L. In one specific example, sodium pyruvate at a concentration of 1 10mgAL is used in the basal medium that is used in the hepatic macrophage culture medium. (11) A manganese supplement, preferably in soluble form. One specific example of such a manganese supplement is manganese (II) chloride. In one specific example, the manganese (II) chloride used is in the form of manganese chloride tetraahydrate (MnCl_{2•}4H₂O). The manganese supplement is usually added in trace amount to the basal medium, for example, at a concentration of between about 1×10⁻⁵ to about 3×10⁻⁴mg/L, or between about 2×10⁻⁵ to about 2.5×10⁻⁴mg/L, or between about 3×10⁻⁵ to about 2×10⁻⁴mg/L, or between about 4×10⁻⁵ to about 1.5×10⁻⁴mg/L, or between about 5×10⁻⁵ to about 1×10⁻⁴mg/L, or between about 6×10⁻⁵ to about 9×10⁻⁵mg/L, or between about 7×10⁻⁵ to about 8×10⁻⁵mg/L, or at about 1×10⁻⁵, 25×10⁻⁵, 5 × 10⁻⁵, 7.5×10⁻⁵, 1×10⁻⁴, 1.5x 10⁻⁴, 2×10⁻⁴, 2.5×10⁻⁴ or 3×10⁻⁴mg/L. In one specific example, manganese (II) chloride at a concentration of 5×10⁻⁵mg/L is used in the basal medium that is used in the hepatic macrophage culture medium. (12) Glucose, a soluble hexose sugar that serves as a primary source of energy for cells. D-Glucose is the natural form used by animal cells. In some examples, the concentration of glucose in the basal medium used in the hepatic macrophage culture is between about 2g/L to about 25g/L, or between about 3g/L to about 22.5g/L, or between about 4g/L to about 20g/L, or between about 5g/L to about 18g/L, or between about 6g/L to about 16g/L, or between about 7g/L to about 14g/L, or between about 8g/L to about 12g/L, or between about 9g/L to about 10g/L, or at about 2.5, 3, 4, 5, 6, 7, 8, 9, 10, 12.5, 15, 17.5, 20, 22.5 or 25g/L. In one specific example, glucose at a concentration of 4.5g/L is used in the basal medium that is used in the hepatic macrophage culture medium. Such a high glucose level is particularly useful in a serum-free medium or low serum medium.

In one specific example, the basal medium used in the hepatic macrophage culture medium is Advanced DMEM medium.

Any cell culture medium may also be supplemented with further components, as and when required based on the experiment to be performed, the cell type in questions, as well as the required status of the cell (starved or otherwise). In some examples, the basal medium is adapted to culture monocytes. In some other examples, the basal medium is adapted to culture hepatocytes. In some further examples, the basal medium is adapated to culture macrophages, in particular hepatic macrophages. The supplements used may depend on the serum level in the cell culture medium. Special supplements may be required when the cell culture medium is a low serum medium or serum free medium. Examples of cell culture supplements that could be used are, but are not limited to, amino acids, chemical compounds, salts, buffering salts or agents, antibiotics, antimycotics, cytokines, growth factors, hormones, lipids, and derivatives thereof.

The basal medium may also contain supplement for cell proliferation, a growth supplement and/or a growth enhancer.

In one example, the growth supplement includes a promoter of glucose and/or amino acid uptake, and/or a promoter of lipogenesis and/or intracellular transport, and/or a promoter of the synthesis of proteins and/or nucleic acids. An example of such a promoter is insulin, in particular human recombinant insulin. Insulin is an important component in low serum or serum free media. Insulin supports cell growth and regulates the cellular uptake and utilization of glucose, amino acids, and lipids. Insulin also has anti-apoptotic properties. In some examples, the insulin in any cell culture medium used herein is at a concentration of between about 0.5 to about 100µg/ml, or between about 1 to about 95µg/ml, or between about 2 to about 90µg/ml, or between about 3 to about 85µg/ml, or between about 4 to about 80µg/ml, or between about 5 to about 75µg/ml, or between about 6 to about 70µg/ml, or between about 7 to about 65µg/ml, or between about 8 to about 60µg/ml, or between about 9 to about 55µg/ml, or between about 10 to about 50µg/ml, or between about 12 to about 45µg/ml, or between about 14 to about 40µg/ml, or between about 16 to about 35µg/ml, or between about 18 to about 30µg/ml, or between about 20 to about 25µg/ml, or at about 0.5, 1, 1.5, 2, 2.5, 3, 3.5, 4, 4.5, 5, 5.5, 6, 6.5, 7, 7,5, 8, 8,5, 9, 9.5, 10, 11, 12, 13, 14, 15, 16, 17, 18, 19, 20, 22, 24, 26, 28, 30, 32, 34, 36, 38, 40, 432, 44, 46, 48, 50, 55, 60, 65, 70, 75, 80, 85, 90, 95 or 100µg/ml, In some specific examples, the insulin in any cell culture medium is at a concentration of between about 1 to about 10µg/ml. In one specific example, the insulin in any cell culture medium is at a concentration of 6.25µg/ml. In some examples, the concentration of insulin in the basal medium used in the hepatic macrophage culture is between about 2mg/L to about 50mg/L, or between about 5mg/L to about 45mg/L, or between about 10mg/L to about 40mg/L, or between about 15mg/L to about 35mg/L, or between about 20mg/L to about 30mg/L, or at about 2, 5, 8, 10, 12.5, 15, 17.5, 20, 22.5, 25, 27.5, 30, 32.5, 35, 37.5, 40, 42.5, 45, 47.5, or 50mg/L. In one specific example, human recombinant insulin (full chain) at a concentration of 10mg/L is used in the basal medium that is used in the hepatic macrophage culture medium.

In another example, the growth supplement is an iron carrier. One example of an iron carrier is transferrin, in particular holo-transferrin. In one specific example, the transferrin is a human transferrin, in particular a human holo-transferrin. In another specific example, the transferrin is a bovine transferrin, in particular a bovine holo-transferrin. Transferrin facilitates extracellular iron storage, and transport. Transferrin may also help to reduce toxic levels of oxygen radicals and peroxide. In some examples, the transferrin in any cell culture medium used herein is at a concentration of between about 0.5 to about 100µg/ml, or between about 1 to about 95µg/ml, or between about 2 to about 90µg/ml, or between about 3 to about 85µg/ml, or between about 4 to about 80µg/ml, or between about 5 to about 75µg/ml, or between about 6 to about 70µg/ml, or between about 7 to about 65µg/ml, or between about 8 to about 60µg/ml, or between about 9 to about 55µg/ml, or between about 10 to about 50µg/ml, or between about 12 to about 45µg/ml, or between about 14 to about 40µg/ml, or between about 16 to about 35µg/ml, or between about 18 to about 30µg/ml, or between about 20 to about 25µg/ml, or at about 0.5, 1, 1.5, 2, 2.5, 3, 3.5, 4, 4.5, 5, 5.5, 6, 6.5, 7, 7,5, 8, 8,5, 9, 9.5, 10, 11, 12, 13, 14, 15, 16, 17, 18, 19, 20, 22, 24, 26, 28, 30, 32, 34, 36, 38, 40, 432, 44, 46, 48, 50, 55, 60, 65, 70, 75, 80, 85, 90, 95 or 100µg/ml, In some specific examples, the transferrin in any cell culture medium is at a concentration of between about 1 to about 10µg/ml. In one specific example, the transferrin in any cell culture medium is at a concentration of 6.25µg/ml. In some examples, the concentration of transferrin in the basal medium used in the hepatic macrophage culture is between about 2.5mg/L to about 37.5mg/L, or between about 5mg/L to about 35mg/L, or between about 7.5mg/L to about 32.5mg/L, or between about 10mg/L to about 30mg/L, or between about 12.5mg/L to about 27.5mg/L, or between about 15mg/L to about 25mg/L, or between about 17.5mg/L to about 22.5mg/L, or at about 2.5mg/L, or about 3mg/L, or about 4mg/L, or about 5mg/L, or about 6mg/L, or about 7mg/L, or about 8mg/L, or about 9mg/L, or about 10mg/L, or about 12mg/L, or about 14mg/L, or about 16mg/L, or about 18mg/L, or about 20mg/L, or about 22mg/L, or about 24mg/L, or about 26mg/L, or about 28mg/L, or about 30mg/L, or about 32mg/L, or about 34mg/L, or about 36mg/L, or about 38mg/L. In one specific example, human holo-transferrin at a concentration of 7.5mg/L is used in the basal medium that is used in the hepatic macrophage culture medium.

In another example, the growth supplement is selenous acid or sodium selenite, which is a co-factor for glutathione peroxidase and other proteins, and is used as an anti-oxidant in cell culture media. In some examples, the selenous acid or sodium selenite in any cell culture medium used herein is at a concentration of between about 0.5 to about 100ng/ml, or between about 1 to about 95ng/ml, or between about 2 to about 90ng/ml, or between about 3 to about 85ng/ml, or between about 4 to about 80ng/ml, or between about 5 to about 75ng/ml, or between about 6 to about 70ng/ml, or between about 7 to about 65ng/ml, or between about 8 to about 60ng/ml, or between about 9 to about 55ng/ml, or between about 10 to about 50ng/ml, or between about 12 to about 45ng/ml, or between about 14 to about 40ng/ml, or between about 16 to about 35ng/ml, or between about 18 to about 30ng/ml, or between about 20 to about 25ng/ml, or at about 0.5, 1, 1.5, 2, 2.5, 3, 3.5, 4, 4.5, 5, 5.5, 6, 6.5, 7, 7,5, 8, 8,5, 9, 9.5, 10, 11, 12, 13, 14, 15, 16, 17, 18, 19, 20, 22, 24, 26, 28, 30, 32, 34, 36, 38, 40, 432, 44, 46, 48, 50, 55, 60, 65, 70, 75, 80, 85, 90, 95 or 100ng/ml. In some examples, the concentration of sodium selenite in the basal medium used in the hepatic macrophage culture is between about 1ng/ml to about 25ng/ml, or between about 2ng/ml to about 22.5ng/ml, or between about 3ng/ml to about 20ng/ml, or between about 4ng/ml to about 17.5ng/ml, or between about 5ng/ml to about 15ng/ml, or between about 7.5ng/ml to about 12.5ng/ml, or at about 2.5ng/ml, or about 5ng/ml, or about 7.5ng/ml, or about 10ng/ml, or about 12ng/ml, or about 14ng/ml, or about 16ng/ml, or about 18ng/ml, or about 20ng/ml, or about 22ng/ml, or about 24ng/ml, or about 25ng/ml. In one specific example, sodium selenite at a concentration of 5ng/ml is used in the basal medium that is used in the hepatic macrophage culture medium.

Insulin, transferrin and selenous acid are commonly used as a growth supplement mixture.

In one example, the growth supplement is a globular non-glycosylated serum protein. One example of such a globular non-glycosylated serum protein is albumin. Bovine serum albumin is commonly used due to its stability and lack of interference within biological reactions. In some examples, the globular non-glycosylated serum protein in any cell culture medium used herein is at a concentration of between about 0.5 to about 50mg/ml, or between about 1 to about 45mg/ml, or between about 1.5 to about 40 mg/ml, or between about 2 to about 35 mg/ml, or between about 2.5 to about 30 mg/ml, or between about 3 to about 25 mg/ml, or between about 3.5 to about 20 mg/ml, or between about 4 to about 15 mg/ml, or between about 4.5 to about 10 mg/ml, or between about 5 to about 9.5 mg/ml, or between about 5.5 to about 9 mg/ml, or between about 6 to about 8.5 mg/ml, or between about 7 to about 8 mg/ml, or at about 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.5, 4, 4.5, 5, 5.5, 6, 6.5, 7, 7.5, 8, 8.5, 9, 9.5, 10, 11, 12, 13, 14, 15, 16, 17, 18, 19, 20, 25, 30, 35, 40, 45 or 50mg/ml. In some specific examples, the globular non-glycosylated serum protein in any cell culture medium is at a concentration of between about 1 to about 5 mg/ml. In one specific example, the globular non-glycosylated serum protein in any cell culture medium is at a concentration of 1.25 mg/ml.

In another example, the growth supplement is fatty acid, in particular polyunsaturated fatty acid essential in biosynthesis of prostaglandins and cell membranes. Examples of such fatty acid include fatty acid of the n-3, n-6 and n-9 families. One example is linoleic acid, which is an n-6 unsaturated fatty acid that is not synthesized by animal cells, and thus needs to be supplemented, especially for serum-free or low serum media. In some examples, the fatty acid in any cell culture medium used herein is at a concentration of between about 0.5 to about 100µg/ml, or between about 1 to about 95µg/ml, or between about 2 to about 90µg/ml, or between about 3 to about 85µg/ml, or between about 4 to about 80µg/ml, or between about 5 to about 75µg/ml, or between about 6 to about 70µg/ml, or between about 7 to about 65µg/ml, or between about 8 to about 60µg/ml, or between about 9 to about 55µg/ml, or between about 10 to about 50µg/ml, or between about 12 to about 45µg/ml, or between about 14 to about 40µg/ml, or between about 16 to about 35µg/ml, or between about 18 to about 30µg/ml, or between about 20 to about 25µg/ml, or at about 0.5, 1, 1.5, 2, 2.5, 3, 3.5, 4, 4.5, 5, 5.5, 6, 6.5, 7, 7,5, 8, 8,5, 9, 9.5, 10, 11, 12, 13, 14, 15, 16, 17, 18, 19, 20, 22, 24, 26, 28, 30, 32, 34, 36, 38, 40, 432, 44, 46, 48, 50, 55, 60, 65, 70, 75, 80, 85, 90, 95 or 100µg/ml, In some specific examples, the fatty acid in any cell culture medium is at a concentration of between about 1 to about 10µg/ml,. In one specific example, the fatty acid in any cell culture medium is at a concentration of 5.35µg/ml.

In another example, the growth supplement is a glutamine supplement. Glutamine supports the growth of cells that have high energy demands and synthesize large amounts of proteins and nucleic acids. It is an alternative energy source for rapidly dividing cells and cells that use glucose inefficiently. Cells require nitrogen atoms to build molecules such as nucleotides, amino acids, amino-sugars and vitamins. Ammonium is an inorganic source of nitrogen that exists primarily as a positively charged cation, NH4+, at physiological pH. Ammonium nitrogen used by cells is initially incorporated into organic nitrogen as an amine of glutamate or an amide of glutamine. These two amino acids provide the primary reservoirs of nitrogen for the synthesis of proteins, nucleic acids and other nitrogenous compounds. Examples of glutamine supplements include L-glutamine and L-alanyl-L-glutamine dipeptide. In some examples, the glutamine supplement in any cell culture medium used herein is at a concentration of between about 0.1% to about 10% mass/volume, or between about 0.2% to about 9.5% mass/volume, or between about 0.3% to about 9% mass/volume, or between about 0.4% to about 8.5% mass/volume, or between about 0.5% to about 8% mass/volume, or between about 0.6% to about 7.5% mass/volume, or between about 0.7% to about 7% mass/volume, or between about 0.8% to about 6.5% mass/volume, or between about 0.9% to about 6% mass/volume, or between about 1% to about 5.5% mass/volume, or between about 1.1% to about 5% mass/volume, or between about 1.2% to about 4.5% mass/volume, or between about 1.3% to about 4% mass/volume, or between about 1.4% to about 3.5% mass/volume, or between about 1.5% to about 3% mass/volume, or between about 1.6% to about 2.5% mass/volume, or between about 1.7% to about 2% mass/volume, or between about 1.8% to about 1.9% mass/volume, or at about 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.25, 2.5, 2.75, 3, 3.25, 3.5, 3.75, 4, 4.25, 4.5, 4.75, 5, 5.5, 6, 6.5, 7, 7.5, 8, 8.5, 9, 9.5 or 10% mass/volume. In some specific examples, the glutamine supplement in any cell culture medium is at a concentration of between about 0.5 to about 2% mass/volume. In one specific example, the glutamine supplement in any cell culture medium is at a concentration of 1% mass/volume.

The buffering agents used to supplement the cell culture medium have to be, in general, chemically and enzymatically stable, having limited effect on biochemical reactions, having high solubility, high membrane impermeability, having very low visible light and ultraviolet light absorbance, and pKa values of between 6.0 and 8.0. One commonly used buffering agent is HEPES buffer ((4-(2-hydroxyethyl)-1-piperazineethanesulfonic acid )), which is a zwitterionic organic chemical buffering agent that is effective in maintaining physiological pH despite changes in carbon dioxide concentration (produced by cellular respiration). In some examples, the buffering agent in any cell culture medium used herein is at a concentration of between about 0.1% to about 10% mass/volume, or between about 0.2% to about 9.5% mass/volume, or between about 0.3% to about 9% mass/volume, or between about 0.4% to about 8.5% mass/volume, or between about 0.5% to about 8% mass/volume, or between about 0.6% to about 7.5% mass/volume, or between about 0.7% to about 7% mass/volume, or between about 0.8% to about 6.5% mass/volume, or between about 0.9% to about 6% mass/volume, or between about 1% to about 5.5% mass/volume, or between about 1.1% to about 5% mass/volume, or between about 1.2% to about 4.5% mass/volume, or between about 1.3% to about 4% mass/volume, or between about 1.4% to about 3.5% mass/volume, or between about 1.5% to about 3% mass/volume, or between about 1.6% to about 2.5% mass/volume, or between about 1.7% to about 2% mass/volume, or between about 1.8% to about 1.9% mass/volume, or at about 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.25, 2.5, 2.75, 3, 3.25, 3.5, 3.75, 4, 4.25, 4.5, 4.75, 5, 5.5, 6, 6.5, 7, 7.5, 8, 8.5, 9, 9.5 or 10% mass/volume. In some specific examples, the buffering agent in any cell culture medium is at a concentration of between about 0.5 to about 2% mass/volume. In one specific example, the buffering agent in any cell culture medium is at a concentration of 1.5% mass/volume.

In one example, the amino acid is, but is not limited to, isoleucine, leucine, lysine, methionine, phenylalanine, threonine, tryptophan, valine, arginine, cysteine, cystine, histidine, tyrosine, alanine, aspartic acid, asparagine, glutamine, glutamic acid, glycine, hydroxproline, proline, serine, combinations and derivatives thereof. In one example, the amino acid is glutamine. The amino acids listed herein may be provided in either the L- or the D-stereoisomer, as required. In one example, the glutamine supplement is L-alanyl-L-glutamine dipeptide. In another specific example, the amino acid is alanine, in particular L-alanine. In some examples, the concentration of L-alanine in the basal medium used in the hepatic macrophage culture is between about 1mg/L to about 50mg/L, or between about 2.5mg/L to about 45mg/L, or between about 5mg/L to about 40mg/L, or between about 7.5mg/L to about 35mg/L, or between about 10mg/L to about 30mg/L, or between about 12.5mg/L to about 27.5mg/L, or between about 15mg/L to about 25mg/L, or between about 17.5mg/L to about 22.5mg/L, or at about 5mg/L, or about 8mg/L, or about 10mg/L, or about 12mg/L, or about 14mg/L, or about 16mg/L, or about 18mg/L, or about 20mg/L, or about 22mg/L, or about 24mg/L, or about 26mg/L, or about 28mg/L, or about 30mg/L, or about 32mg/L, or about 34mg/L, or about 36mg/L, or about 38mg/L, or about 40mg/L, or about 42mg/L, or about 44mg/L, or about 46mg/L, or about 48mg/L, or about 50mg/L. In one specific example, L-alanine at a concentration of 8.9mg/L is used in the basal medium that is used in the hepatic macrophage culture medium.

In one example, the antimycotic is, but is not limited to, amphotericin B, clotimazol, nystatin and combinations thereof.

In a further example, the antibiotic is, but is not limited to, ampicillin, penicillin, chloramphenicol, gentamycin, kanamycin, neomycin, streptomycin, tetracycline, polymyxin B, actinomycin, bleomycin, cyclohexamide, geneticin (G148), hygromycin B, mitomycin C and combinations thereof. In one example, the antibiotic is penicillin. In another example, the antibiotic is streptomycin. In yet another example, the antibiotic is penicillin and streptomycin. In one example, the antibiotic is gentamicin.

In one example, the salt, buffering salt or agent is, but is not limited to, sodium chloride (NaCl), potassium chloride (KCl), sodium hydrogen phosphate (Na₂HPO₄), monosodium phosphate (NaH₂PO₄), monopotassium phosphate (KH₂PO₄), magnesium sulfate (MgSO₄), calcium chloride (CaCl₂), calcium chloride (CaCl₂ × 2 H₂O), sodium bicarbonate (NaHCO₃) and combinations thereof. In one specific example, the buffering agent is sodium bicarbonate (NaHCO₃). In some examples, the concentration of sodium bicarbonate in the basal medium used in the hepatic macrophage culture is between about 0.5g/L to about 20g/L, or between about 1g/L to about 18g/L, or between about 2g/L to about 16g/L, or between about 3g/L to about 16g/L, or between about 4g/L to about 14g/L, or between about 5g/L to about 12g/L, or between about 6g/L to about 10g/L, or between about 7g/L to about 9g/L, or at about 0.5g/L, or about 1.5g/L, or about 2.5g/L, or about 3.5g/L, or about 4.5g/L, or about 5.5g/L, or about 6.5g/L, or about 7.5g/L, or about 8.5g/L, or about 9.5g/L, or about 10.5g/L, or about 11.5g/L, or about 12.5g/L, or about 13.5g/L, or about 14.5g/L, or about 15.5g/L, or about 16.5g/L, or about 17.5g/L, or about 18.5g/L, or about 19.5g/L. In one specific example, sodium bicarbonate at a concentration of 3.7g/L is used in the basal medium that is used in the hepatic macrophage culture medium.

The term "low serum" as used herein refers to a serum level that is lower than what is commonly used. For example, the serum level that is commonly used in cell culture is 10%. Thus, a "low serum" level may refer to the serum level of less than 10%, for example, about 9%, 8%, 7%, 6%, 5%, 4%, 3%, 2%, 1%, 0.5%, 0.1%, or between about 0.1% to about 0.5%, between about 0.5% to about 1.0%, between about 1.0% to about 1.5%, between about 1.5% to about 2.0%, between about 2.0% to about 2.5%, between about 2.5% to about 3.0%, between about 3.0% to about 3.5%, between about 3.5% to about 4.0%, between about 4.0% to about 4.5%, between about 4.5% to about 5.0%, between about 5.0% to about 6.0%, between about 6.0% to about 7.0%, between about 7.0% to about 8.0%, between about 8.0% to about 9.0%, or between about 9.0% to about 9.5%, or between about 9.5% to about 10%. In some specific examples, the low serum level refers to a serum level of 0.1% to about 10%. In some specific examples, the low serum level refers to a serum level of 0.1% to 5%.

In some examples, a serum level of about 0.1% to about 10%, or about 0.1% to about 9%, or about 0.1% to about 8%, or about 0.1% to about 7%, or about 0.1% to about 6%, is used in the basal medium in the method of the first and/or the second aspect during the attachment of the monocytes, for example, during at least the first day, or at least the first 12 hours to 24 hours, or at least the first two days, or at least the first 24 to 36 hours, or at least the first 36 to 48 hours of culturing the monocytes in the hepatic macrophage culture medium. After the monocytes are attached, the serum level in the basal medium can but does not need to be further reduced, or a serum-free basal medium can be used. Thus, in some examples, a serum level of 0% to about 5%, or 0% to about 4%, or 0% to about 3%, or 0% to about 2%, or 0% to about 1%, or 0% to about 0.5%, or 0% to about 0.1%, is used in the basal medium in the method of the first and/or the second aspect after the stem cell-derived monocytes are attached, for example, after the first day, or after the first 12 hours to 24 hours, or after the first two days, or after the first 24 hours to 36 hours, or after the first 36 to 48 hours of culturing the monocytes in the hepatic macrophage culture medium. In some other examples, a serum level of about 0.1% to about 5%, or about 0.1% to about 4%, or about 0.1% to about 3%, or about 0.1% to about 2%, or about 0.1% to about 1%, or about 0.1% to about 0.5%, is used in the basal medium in the method of the first and/or the second aspect after the stem cell-derived monocytes are attached, for example, after the first day, or after the first 12 hours to 24 hours, or after the first two days, or after the first 24 hours to 36 hours, or after the first 36 to 48 hours of culturing the stem cell-derived monocytes in the hepatic macrophage culture medium. In one specific example, after the stem cell-derived monocytes are attached, the basal medium used is serum free.

As described above, the hepatic macrophage culture medium used in the method of the first aspect comprises a conditioned medium and a basal medium. In some examples, the ratio of the conditioned medium to the basal medium is in the range of about 10:1 to about 1: 10, or about 9: 1 to about 1:9, or about 8: 1 to about 1:8, or about 7: 1 to about 1:7, or about 6:1 to about 1:6, or about 5:1 to about 1:5, or about 4:1 to about 1:4, or about 3:1 to about 1:3, or about 2:1 to about 1:2. In some examples, the ratio of the conditioned medium to the basal medium is about 10:1, about 9:1, about 8:1, about 7:1, about 6:1, about 5:1, about 4:1, about 3:1, about 2:1, about 1:1, about 1:2, about 1:3, about 1:4, about 1:5, about 1:6, about 1:7, about 1:8, about 1:9, or about 1:10. In one specific example, the ratio of the conditioned medium to the basal medium is about 1:1.

In specific examples where a low serum basal medium is mixed with a hepatocyte conditioned medium in the ratio of 1:1, and the resulting medium is used to culture monocytes to obtain hepatic macrophages, increased cell differentiation and/or enhanced cell attachment could be observed for the hepatic macrophages obtained.

As used herein, the term "extracellular matrix" or "ECM" refers to a collection of extracellular molecules secreted by cells that provides structural and biochemical support to the surrounding cells. Cell adhesion, cell-to-cell communication and differentiation are common functions of the ECM. Matrix materials may include poly-L-lysine, laminin, gelatin, collagen, keratin, fibronectin, vitronectin, elastin, heparan sulphate, dextran, dextran sulphate, chondroitin sulphate or a mixture of laminin, collagen I, heparan sulfate proteoglycans, and entactin 1, and derivatives or fragment thereof.

The term "feeder cell" as used herein refers to a layer of cells which provides extracellular secretions to facilitate the growth of the stem cells or monocytes. Feeder cells are unable to divide, thus it differs from a co-culture system because only one type of cells (e.g. the stem cells or monocytes) are capable of proliferating. Typical examples of feeder cells are fibroblasts, which are the most common cells in connective tissues. One advantage of a feeder-free cell culture system is that it eliminates the undefined bioactive molecules secreted by feeders.

Also disclosed but not forming part of the present invention is a kit for deriving hepatic macrophages from monocytes, comprising a conditioned medium and a basal medium, wherein the conditioned medium comprises factors secreted by hepatocytes after being cultured in a serum-free cell culture medium in the presence of an extracellular matrix for 1 to 7 days.

The basal medium in the kit may be adapted to be used as a low serum basal medium. The kit can further comprise a cell culture apparatus that is not coated using extracellular matrix. The kit may further comprise any of the following supplements for the basal medium: insulin, transferrin, selenous acid, albumin, fatty acids, glutamine supplements and buffering agent.

The conditioned medium and the basal medium in the kit disclosed herein can be stored at subzero (degree Celsius) temperatures. For example, when the media are stored for long term of more than 6 months, the storage temperature can be about -60°C to about -90°C, or at about -60°C, about -65°C, about -70°C, about -75°C, about -80°C, about -85°C or about -90°C. In one specific example, the media are stored at about -80°C for long-term storage. When stored for short-term usage, the media can be stored at about -10°C to about -30°C, or at about -10°C, about -15°C, about -20°C, about -25°C or about -30°C.

The improved culture media and methods for deriving hepatic macrophages that are provided in the present application will be seen to be applicable to all technologies for which hepatic macrophages are useful. Of particular importance is the use of the culture media and methods as provided herein to derive hepatic macrophages that resemble the characteristics and functions of the primary Kupffer cells, in particular the primary human Kupffer cells. There is limited availability of primary Kupffer cells, especially primary human Kupffer cells, which causes such cells to be expensive. As compared to primary human Kupffer cells, it is easier to scale up the number of hepatic macrophages obtained using the culture media and methods as described herein. For example, hepatic macrophages can be obtained from pluripotent derived monocytes within about 2 weeks, or about 1 week, or about 12 days, about 10 days, about 8 days, about 6 days, or about 4 days using the culture media and methods as described herein. In contrast to primary human Kupffer cells, which cannot be expanded *in vitro,* the hepatic macrophages obtained using the culture media and methods as described herein can be expanded *in vitro.* The culture media and methods as described herein also make it possible for the generation of hepatic macrophages using pluripotent stem cells obtained from the patient, when the hepatic macrophages have to be used together with the hepatocytes obtained from the same patient, such as in co-culturing studies or transplantations. This will help to minimize or eliminate and background immune response present when the hepatocytes and the Kupffer cells are obtained from different subjects.

As used herein, the singular form "a", "an" and "the" include plural references unless the context clearly dictates otherwise. For example, the term "a primer" includes a plurality of primers, including mixtures thereof.

As used herein, the term "about", in the context of concentrations of components of the formulations, typically means +/- 5% of the stated value, more typically +/- 4% of the stated value, more typically +/- 3% of the stated value, more typically, +/- 2% of the stated value, even more typically +/- 1% of the stated value, and even more typically +/- 0.5% of the stated value. When used in the context of duration of time, the term "about" typically means +/- 20% of the stated time, more typically +/- 15% of the stated time, more typically +/- 10% of the stated time, more typically, +/- 5% of the stated time, even more typically +/- 2% of the stated time, and even more typically +/- 1% of the stated time. For example, when the stated duration of time is 1 day, the term "about 1 day" could refer to 1 day +/- 0 to 6 hours. As another example, when the stated duration of time is 1 hour, the term "about 1 hour" could refer to 1 hour +/- 0 to 10 minutes.

Throughout this disclosure, certain examples may be disclosed in a range format. It should be understood that the description in range format is merely for convenience and brevity and should not be construed as an inflexible limitation on the scope of the disclosed ranges. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

The invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non- limiting examples. In addition, where features or aspects of the invention are described in terms of Markush groups, those skilled in the art will recognize that the invention is also thereby described in terms of any individual member or subgroup of members of the Markush group.

### EXPERIMENTAL SECTION

The following examples illustrate methods by which aspects of the invention may be practiced or materials suitable for practice of certain embodiments of the invention may be prepared.

### Example 1 -- Optimization of culture conditions for differentiation of hPSC-KCs

hPSC-derived monocytes (hPSC-Mon) were differentiated from iPSC-IMR90 according to methods described in Wilgenburg *et al.* The initial results showed that embryoid bodies (EBs) could be formed and maintained in culture under serum free and feeder free conditions (**Figure 1A**). EBs adhered within 2 weeks and monocytes could be generated at approximately 18 days of culture. These monocytes could be harvested weekly from the supernatant of the differentiation cultures.

hPSC-Mon were collected from supernatant and differentiated into hepatic macrophages (hPSC-KCs) (**Figure 1A**). hPSC-Mac was used as a control to analyze similarities and differences between non-liver macrophages (NL-Mϕ) and hepatic macrophages (KCs). The results showed that hPSC-Mon could be differentiated into adherent hPSC-KCs (**Figure 1A**).

In order to differentiate hPSC-Mon to hPSC-KCs, hPSC-Mon were treated with culture medium comprising PHHCM combined with Advanced DMEM (low serum) in a 1:1 ratio(**Figure 1A**). The Advanced DMEM contains 0.5% Penicillin/Streptomycin (10,000 U/mL / (10,000 µg/mL), 1X ITS+ containing human recombinant insulin (6.25µg/mL), human transferrin (6.25µg/mL), selenous acid (6.25µg/mL), bovine serum albumin (BSA) - (1.25 mg/mL) and linoleic acid (5.35µg/mL), 2mM GlutaMAX^{™} and 15 mM HEPES buffer. 0.1 to 10% FBS was added to this medium only for cell attachment and reduced to 0 - 5% from the second day of cell culture.

The morphology of hPSC-KCs was compared to PHKCs (**Figure 1B**). The diameter and phenotype of hPSC-KCs was similar to that of PHKCs. Overall the results show that hPSC-KCs could be generated from hPSC-Mon using PHHCM and optimized culture conditions.

### Example 2 -- Marker expression of hPSC-KCs

Following generation of hPSC-KCs, marker expression of the cells was analyzed by gene expression and immunostaining. F4/80 has been documented as a representative marker for mouse Kupffer cells but not for human cells. Recently, CD14 in combination with a classification of CD32, CD68 and CD11 subpopulations of Kupffer cells has been used to define Kupffer cells in humans. In addition, CD 163 has been used as a marker for activated macrophages. Hence, the expression of these markers in hPSC-Mac and hPSC-KC was examined by gene expression studies. The results showed that hPSC-KC expressed CD14, CD163 and CD32 at levels comparable to PHKCs (**Figure 2A**). CD68 and CD11 expression in hPSC-KCs was approximately 30% of PHKCs. The marker expression was confirmed by immunostaining (**Figure 2B**).

### Example 3 -- Cytokine Production by hPSC-KCs upon activation is similar to PHKCs but different to NL-Mϕ

For activation of hPSC-KCs to examine cytokine production, lipopolysaccharide (LPS) was added to the culture medium during the last 16 hours of culture. Medium was collected at the end of the incubation period and morphological changes and cytokine production upon LPS activation was analyzed. The results showed that LPS activation induced typical morphological changes from round to flat and spread cells (**Figure 3A**) in both NL-Mϕ and hPSC-KCs. Importantly, the fold induction in hPSC-KCs was in the same range as that of the PHKCs (25 fold) (**Figure 3B**).. IL-6 production in primary human hepatocytes (PHHs) was below detectable levels. The fold increase in TNF4α production in hPSC-KCs (33 fold) was similar to the fold increase in PHKCs (35 fold) (**Figure 3C**). TNF4α production in PHHs upon LPS activation was below detectable levels. NL-Mϕ produced much higher levels of cytokines compared to PHKCs and hPSC-KCs, which is typical of non-liver macrophages (Figure 3B and Figure 3C).

### Example 4 -- Marker and functional similarities between PHKCs and hPSC-KCs and differences to NL-Mϕ

After confirming that hPSC-KCs expressed macrophage markers and produce cytokines upon LPS stimulation, it was aimed to analyze marker and functional differences between hPSC-KCs and NL-Mϕ. There are limited reports on marker expression differences between macrophages and KCs. CLEC-4F, a member of C-type lectin is the only reported marker which is expressed differentially in Kupffer cells compared to other macrophages. Therefore, it was analyzed whether NL-Mϕ and hPSC-KCs differ in CLEC-4F expression. The results showed that CLEC-4F was expressed in hPSC-KCs and PHKCs, but not in NL-Mϕ (**Figure 4A**). Reports on functional differences between Kupffer cells and macrophages have suggested that Kupffer cells show a higher extent of phagocytosis and lower levels of cytokine production compared to other macrophages. The results on cytokine production already demonstrated that hPSC-KCs produced lower levels of IL-6 and TNF4α compared to NL-Mϕ **(****Figures 3B and 3C**). Next, it was examined if the level of phagocytosis was different in hPSC-Mac and hPSC-KCs. hPSC-KCs, NL-Mϕ and PHKCs were incubated with fluorescent beads for one hour and number of phagocytosed beads were analyzed using a fluorescence microscope. The results showed that all three cell types engulfed the beads (hPSC-KCs and PHKCs, **Figure 4B**; NL-Mϕ, data not shown). Quantification of the beads taken up by the cells showed that hPSC-KCs had a higher percentage of cells which phagocytosed the beads (82%) when compared to NL-Mϕ (54%) (**Figure 4B**). The average number of beads taken up by the cells was also higher in hPSC-KCs when compared to NL-Mϕ (**Figure 4C**). PHKCs showed a slightly lower percentage of cells which phagocytosed the beads (63%) and slightly lower average number of beads uptaken when compared to hPSC-KCs, but these values were still higher than NL-Mϕ. The results indicate that hPSC-KCs and PHKCs are indeed more active in performing phagocytosis compared to NL-Mϕ.

### Example 5 -- Differential toxicity response in hPSC-Mac and hPSC-KCs in co-cultures with PHHs

Previous reports have shown the suppression of CYP expression and activity in human and rat hepatocytes when co-cultured with Kupffer cells. Ethanol induced oxidative stress has been reported to be augmented when hepatocytes were cultured with activated macrophages. However, there are limited reports demonstrating the difference in toxicity responses between human KC-/Mac-hepatocyte co-cultures and hepatocyte monocultures. Brief reports from companies such as Hepregen and Life Technologies have indicated that co-cultures of human hepatocytes and Kupffer cells show differential toxicity responses as compared to hepatocyte monocultures, and might be a more relevant model to mimic hepatotoxic response under inflammation conditions. Therefore, it was analyzed if differences could be identified between PHHs-PHKCs co-cultures and PHHs monocultures in toxicity responses to paradigm hepatotoxicants, and if this difference could be recapitulated in PHHs-hPSC-KCs or PHHs-hPSC-Mac co-cultures. The monocultures and co-cultures were treated with the paradigm hepatotoxicant acetaminophen for 24 hours, and cell viability was measured using Alamar Blue assay. Cell viability was quantified as percentage of cell death compared to vehicle control (DMSO). The results showed that higher cell death was observed in co-cultures of PHHs-PHKCs (**Figure 5A**) and PHHs-hPSC-KCs (**Figure 5B**) when compared to monoculture controls, represented by a typical shift in the toxicity curve to the left. No difference in cell death was observed between PHHs-NL-Mϕ co-cultures and PHHs monocultures (**Figure 5C**). These results suggest that PHHs-KCs might represent a more sensitive model for hepatotoxicity screening, and hPSC-KCs generated in the present study can recapitulate the response shown by commercial PHKCs when co-cultured with PHHs.

## Claims

1. A method of deriving hepatic macrophages from pluripotent stem cells, comprising:
(a) culturing pluripotent stem cells to obtain monocytes; and
(b) culturing the monocytes from (a) in a hepatic macrophage culture medium, wherein the hepatic macrophage culture medium comprises a conditioned medium and a basal medium, wherein the conditioned medium is obtained by a method comprising:
(i) culturing hepatocytes in a serum-free cell culture medium in the presence of an extracellular matrix for 1 to 7 days; and
(ii) isolating the supernatant at the end of the culturing process in (i).

2. The method of claim 1, wherein culturing pluripotent stem cells further comprises:
(i) culturing pluripotent stem cells to obtain embryoid bodies; and
(ii) differentiating embryoid bodies to obtain monocytes.

3. The method of claim 1 or 2, wherein the pluripotent stem cells are induced pluripotent stem cells.

4. The method of any of the preceding claims, wherein the basal medium is a low serum basal medium.

5. The method of any of the preceding claims, wherein culturing the monocytes in a hepatic macrophage culture medium comprises culturing the monocytes in the hepatic macrophage culture medium in the absence of an extracellular matrix.

6. The method of any of the preceding claims, wherein the ratio of the conditioned medium and the basal medium is in the range of 10:1 to 1:10.

7. The method of any of the preceding claims, wherein the ratio of the conditioned medium and the basal medium is 1:1.

8. The method of any one of claims 4 to 7, wherein the serum level in the low serum basal medium is at a percentage of 0.1% to 10%.

9. A method of deriving hepatic macrophages from induced pluripotent stem cells, comprising:
(a) culturing the induced pluripotent stem cells to obtain monocytes; and
(b) culturing the monocytes from (a) in a hepatic macrophage culture medium in the absence of an extracellular matrix, wherein the hepatic macrophage culture medium comprises a conditioned medium and a basal medium, and wherein the conditioned medium is obtained by a method comprising:
(i) culturing hepatocytes in a serum-free cell culture medium in the presence of an extracellular matrix for 1 to 7 days; and
(ii) isolating the supernatant at the end of the culturing process in (i).

## Patentansprüche

1. Verfahren zum Ableiten von hepatischen Makrophagen aus pluripotenten Stammzellen, umfassend:
(a) Kultivieren pluripotenter Stammzellen, um Monozyten zu erhalten; und
(b) Kultivieren der Monozyten aus (a) in einem Kulturmedium für hepatische Makrophagen, wobei das Kulturmedium für hepatische Makrophagen ein konditioniertes Medium und ein Grundmedium umfasst, wobei das konditionierte Medium durch ein Verfahren erhalten wird, das Folgendes umfasst:
(i) Kultivieren von Hepatozyten in einem serumfreien Zellkulturmedium in Gegenwart einer extrazellulären Matrix für 1 bis 7 Tage; und
(ii) Isolieren des Überstands am Ende des Kultivierungsvorgangs nach (i).

2. Verfahren nach Anspruch 1, wobei das Kultivieren pluripotenter Stammzellen ferner Folgendes umfasst:
(i) Kultivieren pluripotenter Stammzellen, um Embryoid-Körper zu erhalten; und
(ii) Differenzieren von Embryoid-Körpern, um Monozyten zu erhalten.

3. Verfahren nach Anspruch 1 oder 2, wobei die pluripotenten Stammzellen induzierte pluripotente Stammzellen sind.

4. Verfahren nach einem der vorhergehenden Ansprüche, wobei das Grundmedium ein Grundmedium mit niedrigem Serumgehalt ist.

5. Verfahren nach einem der vorhergehenden Ansprüche, wobei das Kultivieren der Monozyten in einem Kulturmedium für hepatische Makrophagen ein Kultivieren der Monozyten in dem Kulturmedium für hepatische Makrophagen in Abwesenheit einer extrazellulären Matrix umfasst.

6. Verfahren nach einem der vorhergehenden Ansprüche, wobei das Verhältnis des konditionierten Mediums zu dem Grundmedium im Bereich von 10:1 bis 1:10 liegt.

7. Verfahren nach einem der vorhergehenden Ansprüche, wobei das Verhältnis des konditionierten Mediums zu dem Grundmedium 1:1 beträgt.

8. Verfahren nach einem der Ansprüche 4 bis 7, wobei der Serumspiegel in dem Grundmedium mit niedrigem Serumgehalt bei einem Prozentsatz von 0,1 % bis 10 % liegt.

9. Verfahren zum Ableiten von hepatischen Makrophagen aus induzierten pluripotenten Stammzellen, umfassend:
(a) Kultivieren der induzierten pluripotenten Stammzellen, um Monozyten zu erhalten; und
(b) Kultivieren der Monozyten aus (a) in einem Kulturmedium für hepatische Makrophagen in Abwesenheit einer extrazellulären Matrix, wobei das Kulturmedium für hepatische Makrophagen ein konditioniertes Medium und ein Grundmedium umfasst und wobei das konditionierte Medium durch ein Verfahren erhalten wird, das Folgendes umfasst:
(i) Kultivieren von Hepatozyten in einem serumfreien Zellkulturmedium in Gegenwart einer extrazellulären Matrix für 1 bis 7 Tage; und
(ii) Isolieren des Überstands am Ende des Kultivierungsvorgangs nach (i).

## Revendications

1. Méthode de dérivation de macrophages hépatiques à partir de cellules souches pluripotentes, comprenant :
(a) la culture de cellules souches pluripotentes pour obtenir des monocytes ; et
(b) la culture des monocytes provenant de (a) dans un milieu de culture de macrophages hépatiques, ledit milieu de culture de macrophages hépatiques comprenant un milieu conditionné et un milieu basal, ledit milieu conditionné étant obtenu par une méthode comprenant :
(i) la culture d'hépatocytes dans un milieu de culture cellulaire exempt de sérum en présence d'une matrice extracellulaire pendant 1 à 7 jours ; et
(ii) l'isolement du surnageant à la fin du processus de culture en (i).

2. Méthode selon la revendication 1, ladite culture de cellules souches pluripotentes comprenant en outre :
(i) la culture de cellules souches pluripotentes pour obtenir des corps embryoïdes ; et
(ii) la différenciation des corps embryoïdes pour obtenir des monocytes.

3. Méthode selon la revendication 1 ou 2, lesdites cellules souches pluripotentes étant des cellules souches pluripotentes induites.

4. Méthode selon l'une quelconque des revendications précédentes, ledit milieu basal étant un milieu basal à faible teneur en sérum.

5. Méthode selon l'une quelconque des revendications précédentes, ladite culture des monocytes dans un milieu de culture de macrophages hépatiques comprenant la culture des monocytes dans le milieu de culture de macrophages hépatiques en l'absence d'une matrice extracellulaire.

6. Méthode selon l'une quelconque des revendications précédentes, le rapport entre le milieu conditionné et le milieu basal se trouvant dans la plage de 10:1 à 1:10.

7. Méthode selon l'une quelconque des revendications précédentes, ledit rapport entre le milieu conditionné et le milieu basal étant de 1:1.

8. Méthode selon l'une quelconque des revendications 4 à 7, le taux sérique dans le milieu basal à faible teneur en sérum étant à un pourcentage de 0,1 % à 10 %.

9. Méthode de dérivation de macrophages hépatiques à partir de cellules souches pluripotentes induites, comprenant :
(a) la culture des cellules souches pluripotentes induites pour obtenir des monocytes ; et
(b) la culture des monocytes provenant de (a) dans un milieu de culture de macrophages hépatiques en l'absence d'une matrice extracellulaire, ledit milieu de culture de macrophages hépatiques comprenant un milieu conditionné et un milieu basal, et ledit milieu conditionné étant obtenu par une méthode comprenant :
(i) la culture d'hépatocytes dans un milieu de culture cellulaire exempt de sérum en présence d'une matrice extracellulaire pendant 1 à 7 jours ; et
(ii) l'isolement du surnageant à la fin du processus de culture en (i).
